# EUROPEAN PATENT SPECIFICATION

(11) **EP 3 192 048 B1**
(45) Date of publication and mention of the grant of the patent: **15.03.2023**
(21) Application number: 15839489.0
(22) Date of filing: 10.09.2015
(51) Int. Cl.: G06Q 50/28, G06Q 10/06, G06Q 10/08, G06Q 50/22

(54) **SYSTEM FOR TRACKING UTILIZATION AND CONSUMPTION OF MEDICAL ITEMS IN A MEDICAL FACILITY AND MAINTAINING A CHAIN OF CUSTODY BASED THEREON**
SYSTEM ZUR VERFOLGUNG DER NUTZUNG UND DES VERBRAUCHS MEDIZINISCHER ARTIKEL IN EINER MEDIZINISCHEN EINRICHTUNG UND AUFRECHTERHALTUNG EINER DARAUF BASIERENDEN ÜBERWACHUNGSKETTE
SYSTÈME POUR SUIVRE DE L'UTILISATION ET DE LA CONSOMMATION D'ARTICLES MÉDICAUX DANS UN ÉTABLISSEMENT MÉDICAL ET POUR TENIR À JOUR UNE CHAÎNE DE GARDE SUR LA BASE DE CELUI-CI

(30) Priority: 11.09.2014 US 201462048921 P; 02.10.2014 US 201414504859; 31.12.2014 US 201414587424
(43) Date of publication of application: 19.07.2017
(73) Proprietor: DeRoyal Industries, Inc., Powell, TN 37849 (US)
(72) Inventor: DEBUSK, Brian, C., Knoxville, TN 37922 (US); KAYLOR, Mary, E., Chattanooga, TN 37421 (US); GRIFFITH, Gerald, T., Knoxville, TN 37932 (US); WAGGONER, Timothy, J., Ringgold, GA 30736 (US); GRIFFITH, Jeffrey, D., Knoxville, TN 37917 (US); SEWELL, Angela, M., Knoxville, TN 37924 (US); JACOBS, John, G., Knoxville, TN 37917 (US); HURD, Rex, A., Knoxville, TN 37909 (US)
(74) Representative: Boult Wade Tennant LLP
(86) International application number: PCT/US2015/049373
(87) International publication number: WO 2016/040593

(56) References cited:
- EP-A2- 2 763 075
- WO-A2-2008/024471
- US-A1- 2004 046 020
- US-A1- 2006 176 152
- US-A1- 2008 202 357
- US-A1- 2009 267 772
- US-A1- 2010 079 240
- US-A1- 2010 138 238
- US-A1- 2010 141 457
- US-A1- 2013 060 577
- US-A1- 2013 088 354
- US-A1- 2014 163 726
- US-B1- 6 812 838

## Description

### FIELD

This invention relates to the field of medical item inventory management. More particularly, this invention relates to a system for sensing and recording items that are to be consumed or have been consumed during a medical procedure.

### BACKGROUND

The use of medical supplies and sterile medical devices in the provision of health care services is one of the most significant expenses incurred by most health care facilities. Depending upon the nature and complexity of the medical procedure being performed, a large number of supply items may be used during a medical procedure and, given the priorities of medical personnel involved in the procedure, the ability to track the supplies, gather data about supply utilization and consumption, and record that data in a useable format can be especially difficult. While hospitals and other health care facilities may have sophisticated information systems related to supply inventory management and procedure-based supply requirements, such systems are not able to provide consistent data analysis of supply utilization and optimization if the usage data is not recorded diligently,

In a typical hospital, there are multiple different information systems that are utilized for managing supply inventory and for insuring that the proper supplies are provided for each medical procedure, such as a particular surgery. In the first instance, the hospital supply department will typically have an inventory management system that tracks medical supply inventory, identifies the location of that inventory and records inventory levels as supplies are withdrawn for usage or replaced with new shipmetits of supplies or re-stocks from previously withdrawn but unused supplies. This inventory management system typically tracks the location of supplies in multiple locations throughout the hospital. In some hospitals, this inventory process is still a manual process.

Another common type of information system in a typical hospital that interfaces with the inventory management system is the Operating Room Information System (ORIS). The typical ORIS will provide functionality such as scheduling the operating rooms for procedures, identifying the type of procedure to be performed, identifying the doctor performing the procedure, identifying the assisting nurse(s), and maintaining lists of supplies, devices and instruments (Bills of Materials, or BOM's) that should be provided for each procedure. Typically, these BOM's are specific to (1) the type of procedure being performed and (2) the physician performing the procedure. These BOM's are often maintained in a form known as Doctor Preference Cards.

It is common for the hospital inventory management system to interface with the ORIS in order to insure that the right supplies, devices and instruments are in stock and available for the upcoming scheduled medical procedures. Prior to each case, the BOM for a given procedure and physician is used to pull the appropriate supplies, devices and instruments for that case.

During the case, supply, device and instrument utilization for the procedure should be logged and unused items returned to inventory. When properly logged, useful data about supply utilization is captured and communicated to both the ORIS system and the inventory management system. That data can subsequently be used to capture cost information for the procedure, update the inventory system, prompt necessary re-orders and, as the data for multiple procedures and physicians is accumulated, to analyze supply cost and utilization information for optimization of BOM's to reduce supply waste and identify supply cost savings opportunities.

If accurate information about the consumption of supplies, devices and instruments is not captured, then the ability to identify savings opportunities or to accurately bill for all consumed supply items is lost. It is difficult to insure that this logging step is performed accurately and consistently, since the medical personnel are primarily concerned with insuring the success of the medical procedure. Often, the medical personnel do not have time during the procedure to manually log information into a computer for used items that do not include barcodes, or to scan the barcodes of used items that have barcodes. As a result, much of the information winds up being lost during the turnover of the medical procedure room from one case to another. Another problem with inaccurately recording usage information is the possibility of erroneously charging for items that were not used, which can raise regulatory issues.

The use of RFID tags as part of the inventory control system has potential to facilitate the logging of the supply consumption more accurately and efficiently.
WO 2008/024471 A2 discloses a method for use by a plurality of users to manage and document activities within a laboratory environment using radio frequency identification tags as automated data-entry devices within a Laboratory Information Management System.
US 2009/267772 A1 discloses a payload aware medical cart, system and method that utilizes a computer system to take inventory of the medical products stored in the medical cart.
US 6 812 838 B1 discloses an object control and tracking system and related methods using separate object identification and location detection mechanisms for objects, such as keys, that are maintained in a secure enclosure, such as a key drawer.
EP 2 763 075 A2 discloses a radio-frequency identification reader.
US 2006/176152 A1 discloses a method of RFID power ramping for tag singulation that includes activating the trigger control of an RFID reader for engaging power to begin reading RFID tags.
US 2004/046020 A1 discloses a medication-dispensing unit that is provided for tracking medical products having a Radio Frequency Identification tag uniquely associated therewith.
US 2010/079240 A1 discloses an RFID cabinet for monitoring items having an RFID tag includes a cabinet having at least one locking front door.
US 2013/060577 A1 discloses an inventory management system that manages information regarding medical items dispensed in conjunction with medical treatment of a patient at a medical facility.
US 2014/163726 A1 discloses a group of locking medical storage that are connected together using a daisy-chain connection based on i2C protocol.
US 2010/138238 A1 discloses methods and systems for controlling inventory in a medical facility include providing a medical device into a medical treatment area within a wireless range of a medical facility network and automatically identifying the medical device upon introduction into the medical treatment area.
US 2010/141457 A1 discloses a system and method for tracking supplies, particularly medical supplies, and specifically individual medical items, to the end of the product lifecycle to the point of utilizes.
US 2008/202357 A1 discloses a container having a compactor and bin for waste permitting on-premises and remote monitoring of the system and collection.
US 2013/088354 A discloses a system, method, and device for tracking surgical sponges for a medical procedure.

### SUMMARY

In one aspect, embodiments of the invention and examples use Radio Frequency Identification (RFID) tags to provide the following general functions: (1) identifying medical items or other resources that enter a room or other space in a medical facility; (2) determining where those medical items or other resources came from; and (3) determining whether those medical items or other resources were consumed during a medical procedure performed in the room or space.

In preferred embodiments of the present invention and examples, each item pulled for use during a particular medical procedure in accordance with the Bill of Materials (BOM) for the procedure and the physician includes an RFID tag affixed to the item or the item's outer packaging. These RFID tags contain appropriate inventory information regarding each item as maintained in the inventory control system and the Operating Room Information System (ORIS) or other procedure room software system. Each individual item that might be used can be tracked through use of the RFID tags and appropriate RFID reader technology.

Each Operating Room (OR) or other medical procedure room has a shielded enclosure with one or more RFID antennas disposed inside. A waste bin is disposed in the shielded enclosure. This shielded enclosure and an RFID reader connected to the antennas may be conveniently located near the location where the sterile medical supplies are typically opened by the circulating nurse or other OR personnel responsible for setting up the OR for each procedure, such as near the OR back table. The RFID reader is configured so as to only sense RFID tags that are inside the enclosure and not to sense RFID tags outside the enclosure.

Some preferred embodiments include a portal containing multiple RFID antennas connected to an RFID reader tor reading RFID tags on medial items that are passed through the portal. The RFID reader connected to the portal antennas is preferably configured so as to only sense RFID tags that are inside the portal and not to sense RFID tags outside the portal. Preferably, the portal is also conveniently located near the location where the sterile medical supplies are typically opened by the circulating nurse or other personnel responsible for setting up the room for each procedure. The portal may also be located in areas where supplies are stored outside the procedure room and at other transition locations in the medical facility.

Once the packaging of a medical supply is opened, that item is considered "consumed" because the packaging has been compromised and it cannot be re-stocked. As the packaging of medical supply items having RFID tags are opened, the packaging is dropped into the waste bin inside the shielded enclosure and the reader reads the RFID tags on that packaging. The RFID reader is connected to a data collection interface, such as an ORIS computer terminal, a tablet computer or smart phone, and the consumption information for each item is logged.

This system provides an accurate way to track supply utilization that does not require additional data input steps from the OR personnel. Simply throwing the discarded packaging into a waste bin, which is normal procedure, allows for the RFID tagged supplies to be registered as consumed.

In a further preferred embodiment, a stock bin is provided. Prior to performance of a medical procedure, all RFID-tagged medical supply items that were pulled from the supply room or supply cabinet are placed in the stock bin, the stock bin is moved through the portal or is placed inside the shielded enclosure, and the RFID reader reads the data from the RFID tags on the packaging. In this manner, pre-op data regarding items pulled for use according to a particular BOM can be captured for a given case.

Following the conclusion of the procedure, all RFID-tagged medical supply items that have not been opened, which are thus eligible for re-stocking, are placed into the stock bin, the stock bin is moved through the portal or is placed inside the shielded enclosure, and the RFID reader reads the data from the RFID tags on the packaging. In this manner, post-op data regarding both consumption and non-consumption relative to a given BOM can be captured for a given case. In some embodiments, the RFID reader is connected through a data interface into the ORIS system or the inventory management system and the data regarding the non-consumed items are captured. The process preferably associates medical items (and/or their manufacturer's lot number) and instrument trays to specific patients in the event of a recall or negative occurrence that is determined post-case.

Once the pre-op data and post-op data are accurately collected, the data can be very useful in myriad ways. Since consumption data is accurately determined through the sensing of packaging in the waste bin, billing for medical items consumed in the case can be more accurately reflected on the patient's bill, allowing the hospital to more accurately charge for the procedure. If the stock bin option is included, this ensures that items pulled for the procedure that were detected in the pre-op scan, but were not consumed during the procedure are properly returned to inventory. This process also digitally tracks the movement of each item through various transition locations in the medical facility. This makes it possible to identify excessive handling of items and potential exposures to infectious patients.

More sophisticated data analysis can lead to significant cost improvements, such as by trending consumption and non-consumption for multiple procedures and doctors.

An apparatus for tracking custody of medical items in a supply and consumption chain is provided according to claim 1.

In some embodiments, the medical item inventory database maintains a chain of custody of the medical items in the shipment based on cross-referencing the shipment identification information with the first personnel identifier and the second personnel identifier.

In some embodiments, the apparatus includes a supply room portal having a portal opening and second RFID antennas having fields of view directed to the portal opening. The second RFID antennas receive radio frequency signals emanated from RFID tags attached to medical items that pass through the portal based on the medical items being picked for removal from the supply room to be used in a medical procedure. A second RFID reader that is electrically connected to the second RFID antennas decodes the medical item information contained in the radio frequency signals emanated from the RFID tags. The medical facility inventory computer is programmed to automatically associate a third personnel identifier with the medical item information contained in the radio frequency signals, where the third personnel identifier identifies a person responsible for removal of the medical items from the supply room. This association is triggered by the RFID tags on the medical items in the shipment being detected by the second RFID reader.

In some embodiments, the medical item inventory database maintains the chain of custody of the medical items in the shipment based on cross-referencing the medical item information contained in the radio frequency signals with the third personnel identifier.

In some embodiments, the medical items that have been picked for use in a medical procedure are placed in a transport container. The transport container includes an RFID tag that encodes transport container identification information. In these embodiments, the RFID antennas in the supply room portal receive a radio frequency signal emanated from the RFID tag attached to the transport container as it passes through the portal, and the second RFID reader decodes the transport container identification information. The medical facility inventory computer is programmed to automatically associate the transport container identification information with the medical item information of the medical items disposed in the transport container.

In some embodiments, the apparatus includes a procedure room portal having a portal opening and third RFID antennas having fields of view directed to the portal opening. The third RFID antennas receive radio frequency signals emanated from RFID tags attached to medical items that pass through the procedure room portal when the medical items are brought into a medical procedure room to be used in a medical procedure. A third RFID reader, which is electrically connected to the third RFID antennas, decodes the medical item information contained in the radio frequency signals emanated from the RFID tags. The medical facility inventory computer is programmed to automatically associate a fourth personnel identifier with the medical item information contained in the radio frequency signals, where the fourth personnel identifier identifies a person responsible for the medical items while the medical items are in the medical procedure room. This association is triggered by the RFID tags on the medical items in the shipment being detected by the third RFID reader.

In some embodiments, the medical item inventory database maintains the chain of custody of the medical items in the shipment based on cross-referencing the medical item information contained in the radio frequency signals with the fourth personnel identifier.

the apparatus includes a shielded enclosure disposed in the medical procedure room. The shielded enclosure has an internal space for receiving wrappers of medical items used during the medical procedure and is configured to attenuate radio frequency signals emanated from RFID tags disposed outside the shielded enclosure to levels that are substantially undetectable within the internal space. One or more fourth RFID antennas are disposed within the internal space of the shielded enclosure. The fourth RFID antennas receive radio frequency signals emanated from RFID tags attached to the wrappers disposed within the internal space, which signals contain the medical item information encoded in the RFID tags. A fourth RFID reader, which is electrically connected to the fourth RFID antennas, decodes the medical item information contained in the radio frequency signals emanated from the RFID tags attached to the wrappers disposed within the internal space. The medical facility inventory computer is programmed to automatically associate a patient identifier with the medical item information contained in the radio frequency signals, where the patient identifier identifies a patient on which the medical procedure was performed in the medical procedure room. This association is triggered by the RFID tags of the medical items in the shipment being detected by the fourth RFID reader.

In some embodiments, the medical item inventory database maintains a chain of custody of the medical items in the shipment based on cross-referencing the medical item information contained in the fourth radio frequency signals with the patient identifier.

In some embodiments, the medical facility inventory computer is programmed to automatically generate a fifth message directed to patient treatment personnel if the medical item information indicates that the medical item is a DME medical item. The generation of the fifth message is triggered by the medical item information contained in the radio frequency signals becoming associated with the patient identifier. The fifth message prompts the patient treatment personnel to perform one or more of the following actions: (1) disclose information to the patient related to the proper use of the DME medical item; (2) input information to verify delivery of the DME medical item to the patient; and (3) obtain the patient's signature to acknowledge receipt of the DME medical item.

Some embodiments include a medical facility exit portal having a portal opening and RFID antennas having fields of view directed to the portal opening. The RFID antennas receive radio frequency signals emanated from RFID tags attached to DME medical items that pass through the medical facility exit portal as the DME medical items exit the medical facility in the custody of patients to which the DME medical items have been dispensed. An RFID reader decodes the medical item information contained in the radio frequency signals emanated from the RFID tags attached to the DME medical items. The medical facility inventory computer is programmed to automatically perform one or more of the following actions upon receipt of the medical item information contained in the radio frequency signals:
changing billing records for the patient to indicate that billing for a DME medical item has changed from Medicare Part A, in which the medical facility pays for rental of the DME medical item, to Medicare Part B, in which the patient or the patient's insurance company is billed for the DME medical item;
updating records in the medical item inventory database to transfer custody of the DME medical item from the medical facility to the patient; and
sending notifications to one or more of the patient and medical personnel regarding follow-up care for the patient while using the DME medial item, such notifications including one or more of a recommendation of a Medicare Part B healthcare provider and a prompt for scheduling of follow-up appointments for the patient.

In some embodiments, the RFID antennas of the procedure room portal are operable to receive radio frequency signals emanated from RFID tags attached to medical items that were not used or consumed during the medical procedure and which pass through the procedure room portal as medical items leave the medical procedure room to be returned to the supply room. The medical facility inventory computer of these embodiments is programmed to automatically associate a fifth personnel identifier with the medical item information contained in the radio frequency signals, wherein the association is triggered by the RFID tags on the medical items leaving the medical procedure room. The fifth personnel identifier identifies a person responsible for the medical items until the medical items are returned to the supply room.

In some embodiments, the RFID antennas of the supply room portal are operable to receive radio frequency signals emanated from RFID tags attached to medical items that were not used or consumed during the medical procedure and which pass through the supply room portal as the medical items reenter the supply room to be returned to stock. The medical facility inventory computer of these embodiments is programmed to automatically disassociate the fifth personnel identifier from the medical item information contained in the radio frequency signals, wherein the disassociation is triggered by detection of the RFID tags on the medical items that have been returned to the supply room.

Examples provide an apparatus for maintaining an inventory of medical items in a medical facility, wherein each medical item has an RFID tag attached thereto that encodes medical item information specific to the medical item. The apparatus includes a shielded enclosure comprising shielded walls, a shielded floor, and a shielded ceiling that collectively define an internal space for storing the medical items. A shielded door is disposed in one of the shielded walls of the shielded enclosure and is operable to allow personnel to enter and exit the internal space. A door lock controller electronically controls a lock on the shielded door. One or more sensors associated with the shielded door sense an open state or closed state of the shielded door. One or more storage bins in the internal space hold one or more medical items. RFID antennas in the internal space receive radio frequency signals emanated from the RFID tags attached to the medical items in the storage bins. The radio frequency signals contain the medical item information encoded in the RFID tags attached to the medical items. An RFID reader, which is electrically connected to the RFID antennas, performs scans to detect RFID tags within range of the RFID antennas and decodes the medical item information contained in the radio frequency signals emanated from detected RFID tags. Examples include a computer programmed to discontinue scans by the RFID reader if signals from the one or more sensors associated with the shielded door indicate that the shielded door is in an open state. This prevents the RFID reader from detecting RFID tags that are outside the shielded enclosure.

In some examples, the computer is programmed to cause the door lock controller to maintain the shielded door in a locked state while the RFID reader is performing a scan to detect RFID tags.

In some examples, the computer is programmed to control the RFID reader to perform an RFID system calibration procedure involving a known number of RFID tags attached to medical items disposed in the shielded enclosure. The procedure includes:
(a) performing a scan of the RFID tags with the RFID reader set at a first transmitter power level;
(b) the RFID reader detecting a first number of RFID tags attached to the medical items disposed in the shielded enclosure;
(c) comparing the first number of RFID tags to the known number of RFID tags;
(d) if the first number of RFID tags is less than the known number of RFID tags, performing a scan of the RFID tags with the RFID reader set at a second transmitter power level that is incrementally higher than the first transmitter power level;
(e) repeating steps (b) through (d) until the first number of RFID tags equals the known number of RFID tags; and
(f) operating the RFID reader at the second transmitter power level for ongoing inventory maintenance purposes.

In some examples, the calibration procedure includes:
(a) performing a scan of the RFID tags with the RFID reader set at a first receiver sensitivity level;
(b) the RFID reader detecting a first number of RFID tags attached to the medical items disposed in the shielded enclosure;
   € comparing the first number of RFID tags to the known number of RFID tags;
(d) if the first number of RFID tags is less than the known number of RFID tags, performing a scan of the RFID tags with the RFID reader set at a second receiver sensitivity level that is incrementally higher than the first receiver sensitivity level;
   € repeating steps (b) through (d) until the first number of RFID tags equals the known number of RFID tags; and
(f) operating the RFID reader at the second receiver sensitivity level for ongoing inventory maintenance purposes.

### BRIEF DESCRIPTION OF THE DRAWINGS

Other embodiments of the invention will become apparent by reference to the detailed description in conjunction with the figures, wherein elements are not to scale so as to more clearly show the details, wherein like reference numbers indicate like elements throughout the several views, and wherein:
FIG. 1 depicts a system for sensing and recording consumption of medical items during a medical procedure;
FIGS. 2A and 2B depict shielded enclosures;
FIG. 3 depicts a method for sensing and recording consumption of medical items during a medical procedure;
FIG. 4 depicts a method for programming RFID tags for use on medical items;
FIGS. 5 A-5C depict display screens displayed to a user of the system while performing the method depicted in FIG. 4;
FIG. 6 depicts a method for programming RFID tags for use on storage bins used for carrying medical items;
FIGS. 7A-7C depict display screens displayed to a user of the system while performing the method depicted in FIG. 6;
FIG. 8 depicts a method for reading RFID tags on medical items placed in the shielded enclosure;
FIGS. 9A-9C depict display screens displayed to a user of the system while performing the method depicted in FIG. 8;
FIG. 10 depicts a method for reading RFID tags on medical items passed through a portal;
FIGS. 11A-11B depict display screens displayed to a user of the system while performing the method depicted in FIG. 10;
FIG. 12 depicts a method for searching for medical items having RFID tags that have been scanned into the system;
FIG. 13 depicts a method for searching for medical items and retrieving item data;
FIG. 14 depicts a method for system maintenance;
FIG. 15 depicts a display screen displayed to a user of the system while performing the method depicted in FIG. 12;
FIG. 16 depicts a display screen displayed to a user of the system while performing the method depicted in FIG. 13;
FIG. 17 depicts a display screen displayed to a user of the system while performing the method depicted in FIG. 14;

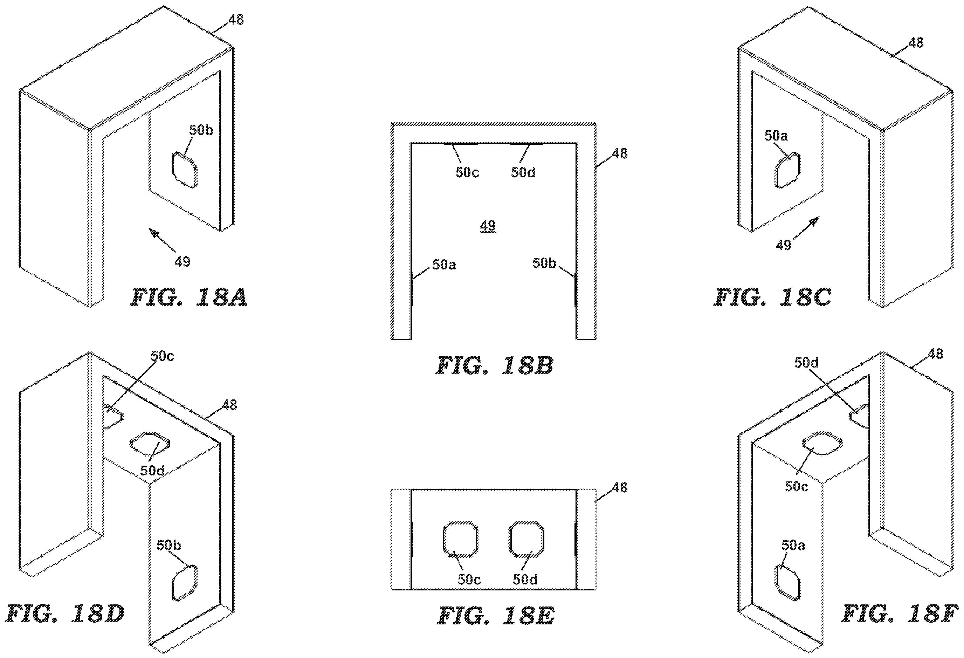

FIGS. 18A-18F depict a portal;
FIGS. 19 and 20 depict processes for sensing and recording utilization of medical resources in the performance of a medical procedure in a medical facility;
FIGS. 21 and 22 depict processes for generating alerts based on utilization of medical resources in the performance of a medical procedure in a medical facility;
FIGS. 23, 24A, 24B and 25 depict a system for tracking medical items through various transition points in a supply and consumption chain;
FIG. 26 depicts an apparatus for maintaining an inventory of medical items;
FIG. 27 depicts a computer system associated with a supply room door;
FIGS. 28 and 29 depict exemplary user interface screens generated by a RFID label printer application;
FIG. 30 depicts an exemplary printed RFID label; and
FIGS. 31 and 32 depict exemplary user interface screens generated by the RFID label printer application.

### DETAILED DESCRIPTION

As the term is used herein, a "medical item" is an item, material, substance, or piece of durable medical equipment (DME) that is used or consumed during the performance of a medical procedure or that is dispensed to a patient to treat a medical condition or provide comfort to the patient. For example, sponges, gloves and drapes are medical items. A surgical implant is another example of a medical item. Knee braces, negative pressure wound therapy units, blood glucose monitors, and wheelchairs are further examples of medical items. Medical items comprise a subset of "medical resources," As the term is used herein, a "medical resource" is any item, person, piece of equipment, or space involved in providing medical services for a patient. For example, a gurney on which a patient lies during a surgical procedure is a medical resource. The doctor performing the procedure, the attending nurses, and the patient are also medical resources. An operating room is a medical resource.

As the term is used herein, a "wrapper" encompasses all manner of containers and packaging, sterile or non-sterile, in which a medical item is or has been enclosed. The term "wrapper" also includes a label, hang tag, or other such device that may be attached to a medical item without completely enclosing the item. The term "wrapper" further includes packaging for a sterile-wrapped kit of medical items, such as a tray of implants and supplies for use in a surgical procedure, wherein an RFID tag is attached to the tray. Generally, anything that may function to associate an RFID tag with a medical item is encompassed by the term "wrapper."

Each medical item has a unique item identifier encoded in a machine-readable code in an RFID tag, a QR code, a bar code, or a combination thereof attached to the medical item or its wrapper. In some embodiments, an RFID tag and a QR code are combined in a single label applied to the medical item or its wrapper.

In a preferred embodiment, each wrapper includes an RFID tag attached thereto or embedded therein. Ultra High Frequency (UHF) passive RFID tags are preferred for this application, as they may be interrogated from up to about 30 centimeters to about 30 feet (9.144 metres) away. In preferred embodiments, each RFID tag is encoded with a unique item identification number for the particular medical item associated with the wrapper. An item information database 52 associates each item identification number with item-specific information, such as the manufacturer part number, item description, vendor, cost, Latex content, expiration date, and inventory location. Additionally or alternatively, the RFID tag may be encoded with item-specific information as set forth in Unique Device Identification (UDI) standards set by the U.S. Food and Drug Administration (FDA).

In some embodiments, item-specific information encoded in RFID tags on medical items may be used to generate alerts for medical personnel. For example, an alert may be generated if information encoded in an RFID tag indicates the presence of Latex in an item, and the patient is allergic to Latex, Also, an alert may be generated if information encoded in an RFID tag indicates that an item's useful lifetime has expired or if the item is from a lot that has been recalled by the manufacturer.

As the term is used herein, a "portal" is any passageway, opening, aperture, window, panel, wall, doorway, hallway, pathway, or aisle in or near which one or more RFID antennas are mounted for sensing RFID tags that pass through or near the portal. A portal may also be a handheld scanning device for reading RFID tags. Several portals may be used to track the routes of travel and locations of medical resources throughout a medical facility or a medical item supplier facility.

As the term is used herein, a "scan" for RFID tags refers to operations performed by an RFID reader to transmit signals and receive signals from RFID tags that are in range of the RFID reader and its associated antenna(s).

In preferred embodiments, portals are placed at "transition locations" within a medical facility or a medical item supplier facility. Examples of transition locations include supply rooms, supply cabinets, procedure rooms, waste containers, personnel break rooms, hallways, shipment assembly areas, shipment loading docks, and points of entry into and exit from the medical facility or a medical item supplier facility.

As the term is used herein, a "shipment" of medical items comprises multiple medical items, of the same type or different types, that are packaged together at a supplier location and shipped to a location at which the medical items are consumed or dispensed. Generally, each shipment includes a packing list that lists all of the medical items in the shipment. Each packing list has a unique shipment identifier that encodes shipment identification information that is specific to the shipment. The shipment identifier may be in the form of an RFID tag, bar code, or other encoded identifier attached to, embedded in, or printed on the packing list.

### Sensing and Logging Consumption of Medical Items During Medical Procedure

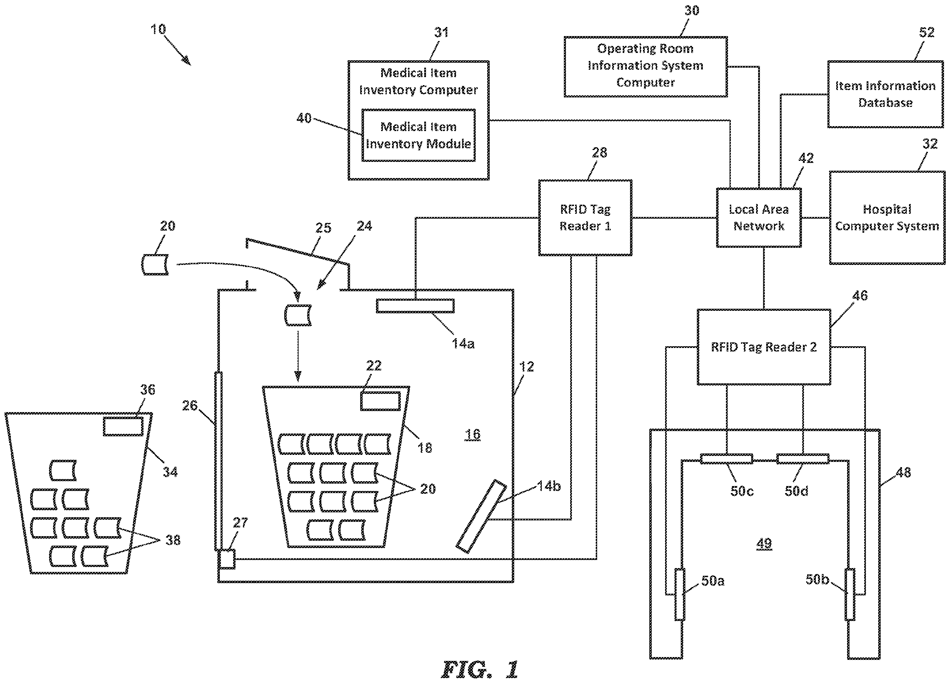

As shown in FIG. 1, a system 10 for sensing and logging consumption of medical items during a medical procedure includes a shielded enclosure 12 having a space 16 that is large enough to receive a waste bin 18. Disposed within the enclosure 12 are two RFID antennas 14a and 14b, such as Laird 5 x 5 inch (12.7 centimetres x 12.7 centimetres) Mini Far Field antennas (model number S9025PLNF) having left-hand circular polarization and operating in the 902-928 MHz frequency range. In an alternative embodiment, a single larger Laird antenna is used. One of the antennas 14a is preferably disposed at the top of the enclosure 12, with its field of view looking downward into the space 16. The other RFID antenna 14b is preferably disposed at the bottom of the enclosure 12, with its field of view looking upward into the space 16. The RFID antennas 14a-14b are electrically connected, such as via a coaxial cable, to a UHF RFID tag reader 28. In a preferred embodiment, the RFID tag reader 28 is an Impinj^{®} Speedway^{®} model R420.

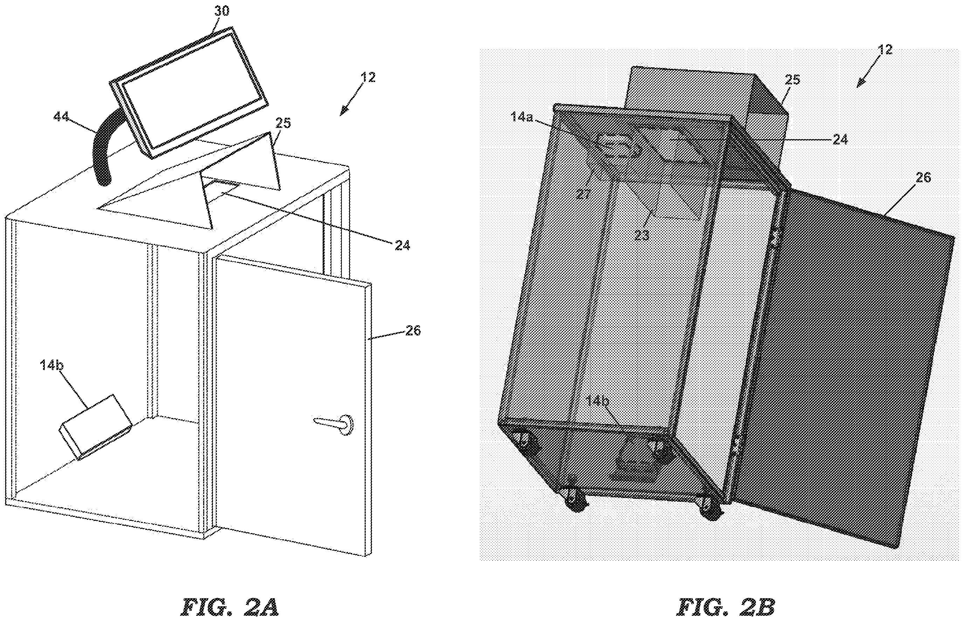

Preferred embodiments of the shielded enclosure 12 are shown in FIGS. 2A and 2B, wherein the sidewalls are depicted as transparent. The enclosure 12 is preferably made from 0.080 inch (2.032 millimetres) thick sheet aluminum supported by 0.75 x 0.75 inch (1.905 centimetres x 1.905 centimetres) square aluminum tubing (0.125 inch (3.175 millimetres) thick). The outside dimensions of the preferred embodiment are 23.5 x 22.0 x 40.75 inches (59.69 centimetres x 55.88 centimetres x 103.505 centimetres).

As the term is used herein, "shielded" means that the enclosure 12 is designed to prevent the antennas 14a-14b from receiving RFID signals from RFID tags located outside the enclosure 12 at a signal-to-noise ratio high enough to trigger detection of those outside RFID tags. For purposes of this disclosure, "shielded" does not mean that absolutely all RF energy is blocked from entering the enclosure, as this would require unnecessary levels of shielding.

An opening 24 is provided in the top of the enclosure that is large enough to receive wrappers or containers 20 from which medical items have been removed. The opening 24 is preferably a 6.75 x 13.75 inch (17.145 centimetres x 34.925 centimetres) rectangle. An aluminum cover 25 is provided over the opening 24. The cover may be slanted as shown in FIG. 2A or more box-like as shown in FIG. 2B to prevent signals from escaping the enclosure 12. As shown in FIG. 2B, the enclosure preferably includes an aluminum chute 23 around the opening 24, and an aluminum shield 27 around the antenna 14a. These structures provide further attenuation of RFID signals originating outside the enclosure 12 to prevent those signals from being detected by the antennas 14a-14b. The waste bin 18 is positioned below the opening 24 so that wrappers 20 deposited in the opening 24 fall into the bin 18. In a preferred embodiment, a hinged door 26 large enough to receive the waste bin 18 is provided in a sidewall of the enclosure 12. The door 26 is preferably 29.5 x 39.25 inch (74.93 centimetres x 99.695 centimetres), and includes a handle/latch for securing the door in a closed position. The enclosure 12 is considered to be shielded when the door 26 is closed.

In a preferred embodiment, the system 10 includes a portal 48 having an opening 49 at least large enough to receive the waste bin 18. The portal 48 is preferably equipped with four RFID antennas 50a-50d having fields of view looking inward into the portal opening 49. The RFID antennas 50a-50d are electrically connected, such as via coaxial cables, to a UHF RFID tag reader 46. In a preferred embodiment, the RFID tag reader 46 is an Impinj^{®} Speedway^{®} model R220. In some embodiments, the tag reader 46 and the tag reader 28 comprise a single tag reader.

The waste bin 18, also referred to herein as a waste tote, is preferably a plastic container having an open top for receiving wrappers 20. In some embodiments, an RFD tag 22 encoded with a unique bin identification number is attached to the waste bin 18. The database 52 associates the bin identification number with a particular procedure room to which the waste bin 18 is assigned. Alternatively, the RFID tag 22 may be encoded with information indicating the procedure room to which the bin 18 is assigned.

The RFID tag readers 28 and 46 are electrically connected via a local area network (LAN) 42 to a medical item inventory computer 31, which may be a server computer, desktop computer, laptop computer, tablet computer or other mobile computing device. Alternatively, the electrical connection between the RFID tag readers 28 and 46 and the computer 31 is via a Universal Serial Bus (USB) interface. The computer 31 includes memory for storing and a processor for executing instructions of a medical item inventory module 40. In preferred embodiments, the medical item inventory module 40 compiles pre-op and post-op lists of items, compares the lists to detect discrepancies, generates alert messages upon detection of discrepancies, and updates inventory records based on actual item usage.

In a preferred embodiment, an Operating Room Information System (ORIS) computer 30 is in communication with the medical item inventory computer 31 via a communication network, such as the LAN 42. The ORIS computer 30 is also in communication with a hospital computer system 32 via a communication network, such as the LAN 42. In preferred embodiments, the hospital computer system 32 manages medical item inventories, procedure room scheduling, patient records, insurance reimbursement/payment functions, and admission/discharge/transfer (ADT) records. The hospital computer system 32 may also include or be connected to an electronic data interchange server, such as a J.D. Edwards/Oracle server, that implements electronic commerce transactions between the hospital and medical item suppliers.

In some embodiments, the medical item inventory module 40 is a software application running on the computer 31. In alternative embodiments, the medical item inventory module 40 is executed by a remote computer (outside the OR). For example, the medical item inventory module 40 may be implemented as "software-as-a-service" provided via the Internet by a medical item inventory service provider.

With continued reference to FIG. 1, a preferred embodiment of the system 10 includes a stock bin 34, which may also be referred to herein as a transport bin, transport container, or stock tote. As described in more detail below, the stock bin 34 is used to transfer medical items 38 to be used during a medical procedure from a stock room to the procedure room, and to transfer unused medical items 38 from the procedure room back to the stock room. In some embodiments, an RFID tag 36 is attached to the stock bin 34 that is encoded with a unique bin identification number. In some embodiments, the database 52 associates the bin identification number with a particular procedure room or stock room to which the stock bin 34 is assigned. Alternatively, the RFID tag 36 may be encoded with information indicating the procedure room or stock room to which the stock bin 34 is assigned.

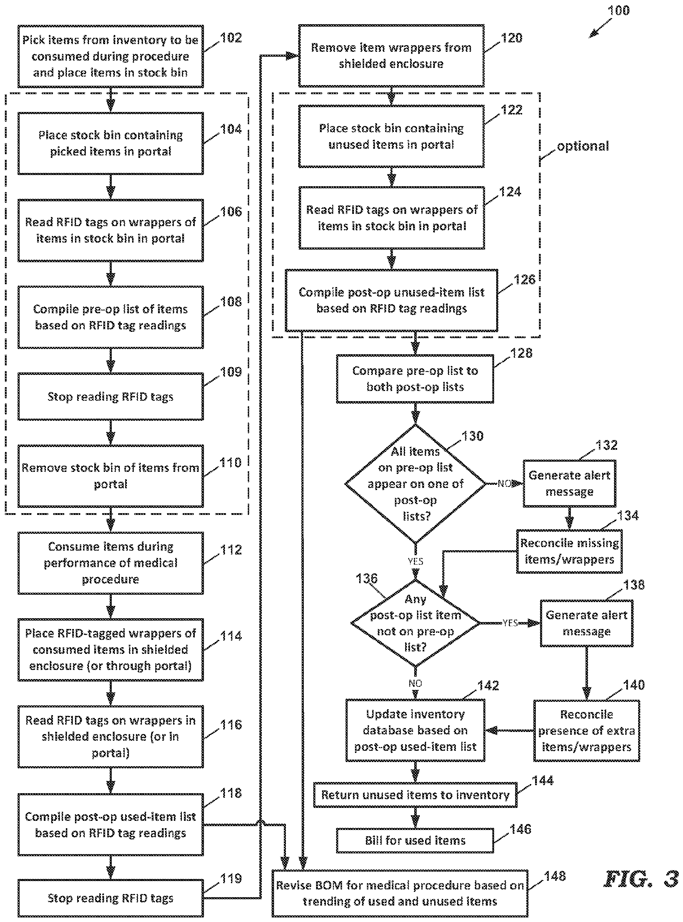

FIG. 3 depicts an example of a process 100 for sensing and recording consumption of medical items during a medical procedure using the system depicted in FIG. 1. To begin the process, hospital personnel pick medical items from inventory stock to be used during the medical procedure (step 102 in FIG. 3). For example, the needed items may be listed on a Bill of Materials (BOM) for the particular type of procedure to be performed. In some cases, the BOM also reflects the individual preferences of particular doctors. These types of BOM's may also be referred to as Doctor Preference Cards. The picked items are placed in the stock bin 34 to be transferred to the OR.

In one example, the stock bin 34 containing the picked items 38 is placed in or passed through the portal 48 outside the procedure room (step 104) and the RFID reader 46 reads the RFID tags on the wrappers of the items 38 in the stock bin 34 (step 106). In some examples, activation of the reader 28 is triggered manually by a person in the procedure room using an interface device (mouse, touchpad or keyboard) of the computer 31.

The item identification numbers read from the RFID tags in the portal 48 are transferred to the medical item inventory computer 31 where the medical item inventory module 40 compiles a pre-op list of the items 38 in the stock bin 34 (step 108). In an example, the date/time of the compilation of the list is recorded in the medical item inventory computer 31, along with the identification number of the stock bin 34. Other information may be associated with the pre-op list, such as procedure room number, doctor name, patient name, patient age, patient weight, patient allergies, type of medical procedure, and case number. Once the pre-op list is compiled, the RFID reader 28 may be deactivated (step 109) and the stock bin 34 removed from the portal 48 (step 110).

Steps 104-110 of FIG. 3 are optional and are not implemented in all examples of process 100. If these steps are not performed, the BOM for the medical procedure may serve the purpose of the pre-op item list.

The items 38 are preferably removed from the bin 34 and arranged on a table in the procedure room according to the doctor's or attending nurse's preference. As the items 38 are used/consumed during the procedure (step 112), wrappers 20 removed from the items 38 are dropped through the opening 24 in the enclosure 12 where they are received into the waste bin 18 (step 114). When the wrappers 20 enter the enclosure 12, the RFID tags on the wrappers 20 are detected and read by the reader 28 (step 116).

The item identification numbers read from the RFID tags in the enclosure 12 are transferred to the medical item inventory computer 31 where the medical item inventory module 40 compiles a post-op used-item list of the wrappers 20 (step 118). In an example, the date/time that each wrapper 20 was first detected is recorded in the list. Also, the identification number of the waste bin 18 (if any) and other information may be associated with the post-op used-item list, such as procedure room number, doctor name, patient name, type of medical procedure, and case number. Once the post-op used-item list is compiled, the RFID reader 28 is deactivated (step 119) so that it will not read any other tags when the door 26 is opened to remove the wrappers 20 (step 120). Deactivation of the reader 28 may be triggered by opening the door 26 of the enclosure 12.

In an alternative example, the waste bin 18 remains outside the shielded enclosure 12 during the procedure. As the items 38 are used/consumed during the procedure (step 112), wrappers 20 removed from the items 38 are deposited in the waste bin 18. After completion of the procedure, the waste bin 18 containing the wrappers 20 is placed through the portal 48 (step 114), and the reader 28 reads the RFID tags of the wrappers 20 (step 116). The post-op used-item list is compiled as described in the previous example (step 118).

In some examples, after completion of the medical procedure, all unused items 38 are placed back into the stock bin 34, and the stock bin 34 is passed through the portal 48 (step 122). The reader 46 reads the RFID tags of the unused items 38 (step 124), and a post-op unused-item list is compiled (step 126). The identification number of the stock bin 34 and other information may be associated with the post-op unused-item list, such as procedure room number, doctor name, patient name, type of medical procedure, and case number.

Steps 122-126 of FIG. 3 are optional and are not implemented in all examples of process 100. If these steps are not performed, the post-op unused-item list may be generated by comparing the BOM to the post-op used item list.

Various examples use the pre-op and post-op item lists to implement various advantageous inventory and billing functions. For example, the medical item inventory module 40 may compare the items listed in the pre-op list to the items listed in the post-op used-item list and the post-op unused-item list (step 128). If an item in the pre-op list does not appear on either of the post-op lists (step 130), this means the item was brought into the procedure room but neither the item nor its wrapper ended up in the stock bin or the waste bin after the procedure. In this case, an alert is generated that causes a message to appear on a display screen of the OBIS computer 30 or the medical item inventory computer 31 (step 132). The alert should prompt the procedure room personnel to investigate three possibilities that may have caused the discrepancy: (1) the item is unused and still in the procedure room but was inadvertently not placed back into the stock bin before the post-op unused-item list was compiled, (2) the item was used and its wrapper is still in the procedure room but the wrapper was inadvertently not placed in the waste bin before the post-op used-item list was compiled, or (3) the item and/or its empty wrapper was removed from the procedure room prior to compilation of either of the post-op lists. In any event, the missing item(s) or wrapper(s) should be located and the pre-op and post-op lists reconciled (step 134).

If the comparison of the pre-op and post-op item lists indicates that an item that appears on either of the post-op lists is not on the pre-op list (step 136), this means that the item or its wrapper was present in the procedure room when the post-op lists were compiled, but it was (1) not brought into the procedure room in the stock bin with the other items, or (2) brought into the procedure room in the stock bin but was removed from the stock bin prior to compilation of the pre-op list. In this case, an alert is generated which causes a message to appear on a display screen of the computer 31 (step 138). The alert should prompt the procedure room personnel to investigate what may have caused the discrepancy and reconcile the pre-op and post-op lists (step 140).

In an example, once the post-op lists are complete and reconciled, the computer 31, the ORIS computer 30, or the hospital computer system 32 uses the lists to update the database 52 based on actual item usage (step 142). The hospital computer system 32 or the ORIS computer 30 also may use the post-op used-item list to accurately bill the patient (or insurance company) for the items used during the procedure (step 146). The stock bin 34 may be returned to the appropriate inventory stock room where the unused items 38 may be returned to inventory (step 144).

In examples, the hospital computer system 32 or the Medical Item Inventory Application 40 analyzes the post-op unused-item lists generated during multiple procedures of the same type and for the same doctor to determine trends in the lack of usage of certain medical items that are listed on BOM's (step 146). This trend data may be used to revise the BOM's for certain procedures/doctors. For example, if the trend data indicates that in 90% of hip replacement surgeries performed by Dr. Jones only three sponges of a particular type are used out of the five called for on the BOM, the BOM may be revised to call for only three sponges. Revisions of this sort would reduce the effort/cost associated with returning unused items to the stock room, and would decrease traffic in and out of the procedure room during a procedure which would decrease the chances of a site infection. Trend data may also be used to determine the optimal locations to store medical supplies and the optimal quantities to store.

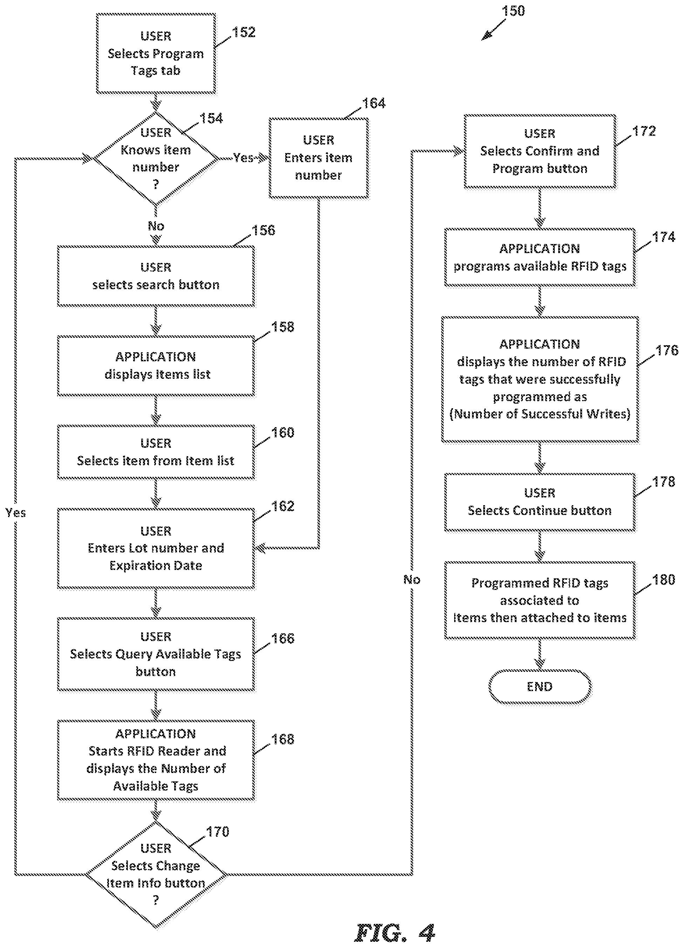

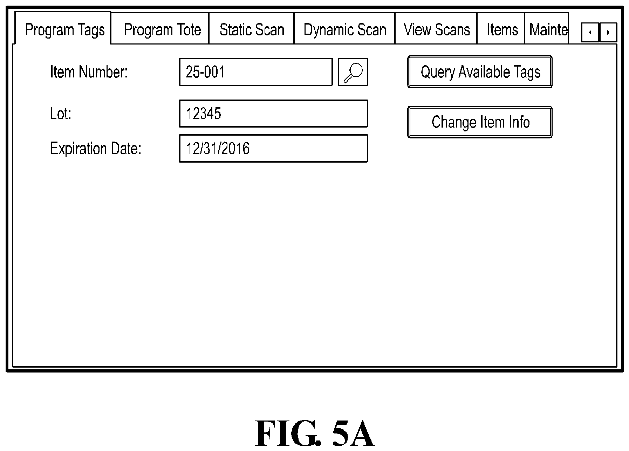

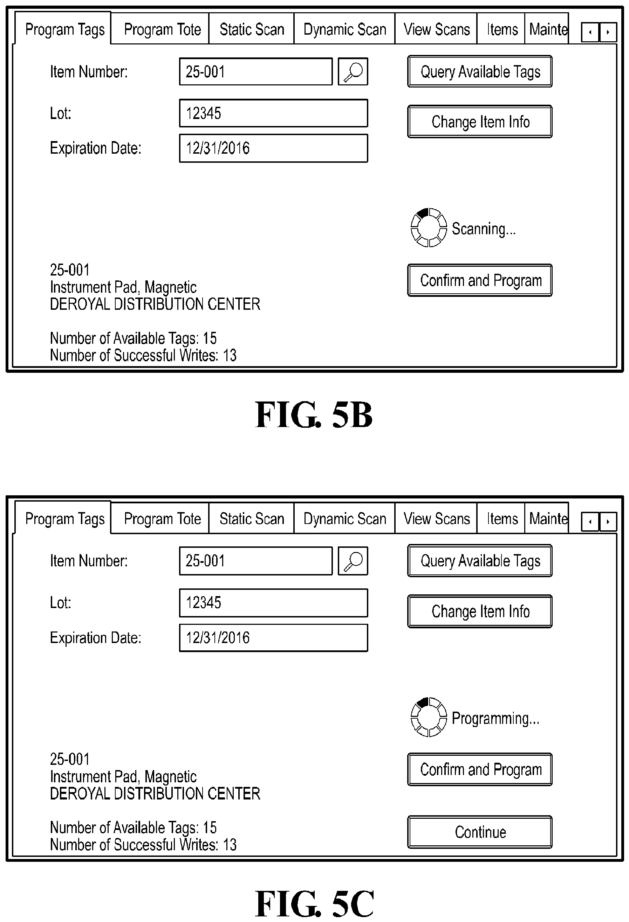

FIG. 4 depicts an example of a method 150 for programming RFID tags for medical items. While running the medical item inventory application, the user selects the "Program Tags" tab on the example display screen depicted in FIG. 5A (step 152). If the user does not know the item number of the medical item for which a tag is to be programmed (step 154), the user may select the "Search" button (step 156). This causes the application to display an items list (step 158) from which the user selects the item (step 160). The user then enters the lot number and expiration date (step 162) and selects the "Query Available Tags" button (step 166). This activates the RFID reader/writer to detect and display a number of tags that are available for programming (step 168). In the example of FIG. 5B, the RFID reader/writer detected fifteen tags available for programming. Before programming the tags with item information, the user has an opportunity to edit the item information (step 170). If the item information is complete and accurate, the user selects the "Confirm and Program" button (step 172). This causes the RFID reader/writer to program the available RFID tags with the item information (step 174). The number of tags that are successfully programmed are indicated as "Number of Successful Writes" as shown in FIG. 5C (step 176). The user then selects the "Continue" button (step 178), which causes the application to associate the newly programmed tags with the item number in the database 52 (step 180).

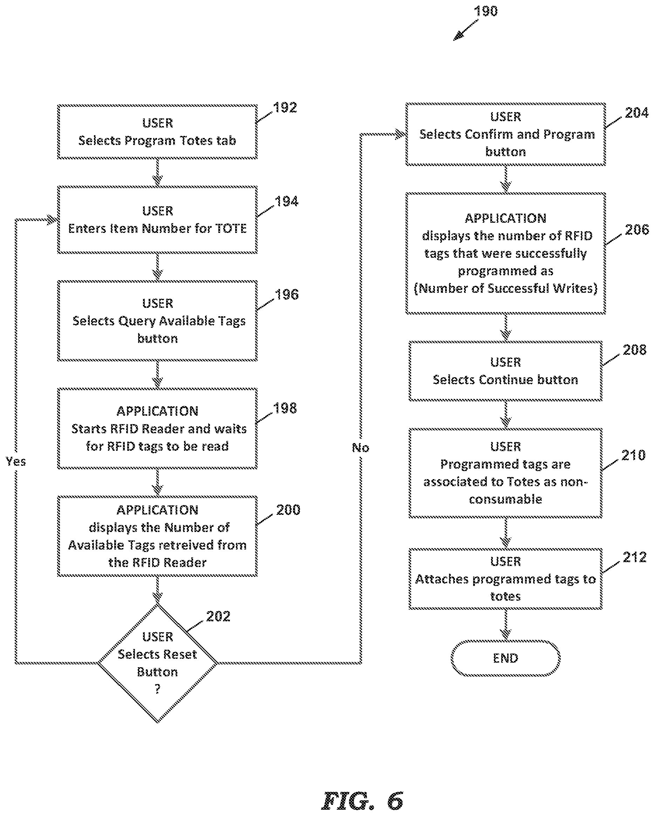

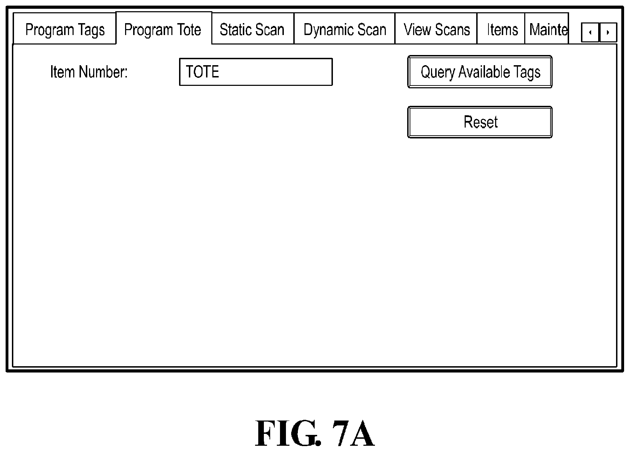

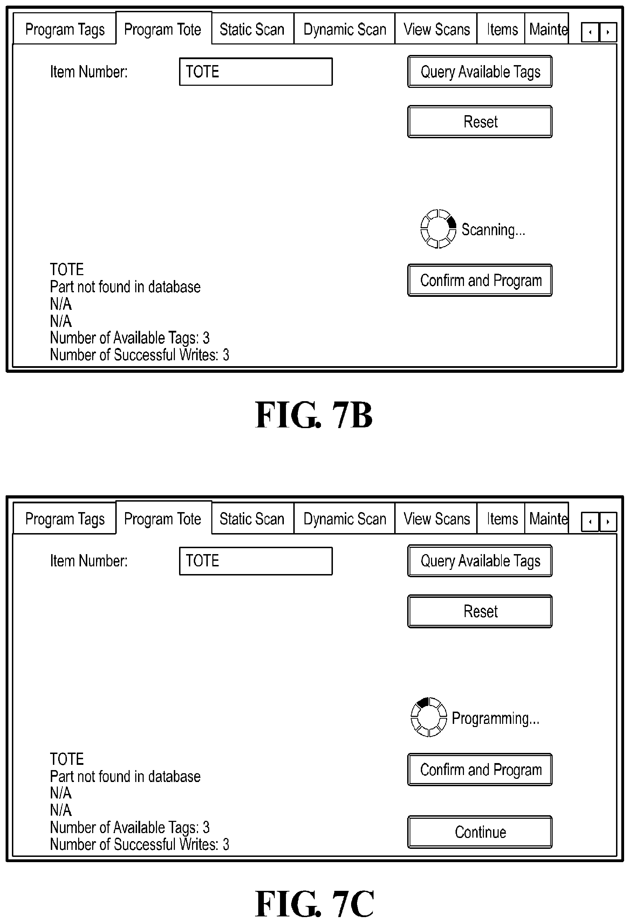

FIG. 6 depicts an example of a method 190 for programming RFID tags for bins or totes, such as the waste bin 18 or the storage bin 34. While running the medical item inventory application, the user selects the "Program Totes" tab on the example display screen depicted in FIG. 6A (step 192). The user then enters the item number for the tote (step 194) and selects the "Query Available Tags" button (step 196). This activates the RFID reader/writer to detect the number of tags that are available for programming (step 198) and display the available number on the display device (step 200). In the example of FIG. 7B, the RFID reader/writer detected three tags available for programming. If the user wishes to proceed with the programming process, the user selects the "Confirm and Program" button (step 204). This causes the RFID reader/writer to program the available RFID tags with the tote information (step 206). The number of tags that are successfully programmed are indicated as "Number of Successful Writes" as shown in FIG. 7C (step 176). The user then selects the "Continue" button (step 208), which causes the application to associate the newly programmed tags with non-consumable totes in the database 52 (step 210). The programmed tags are then attached to the totes (step 212).

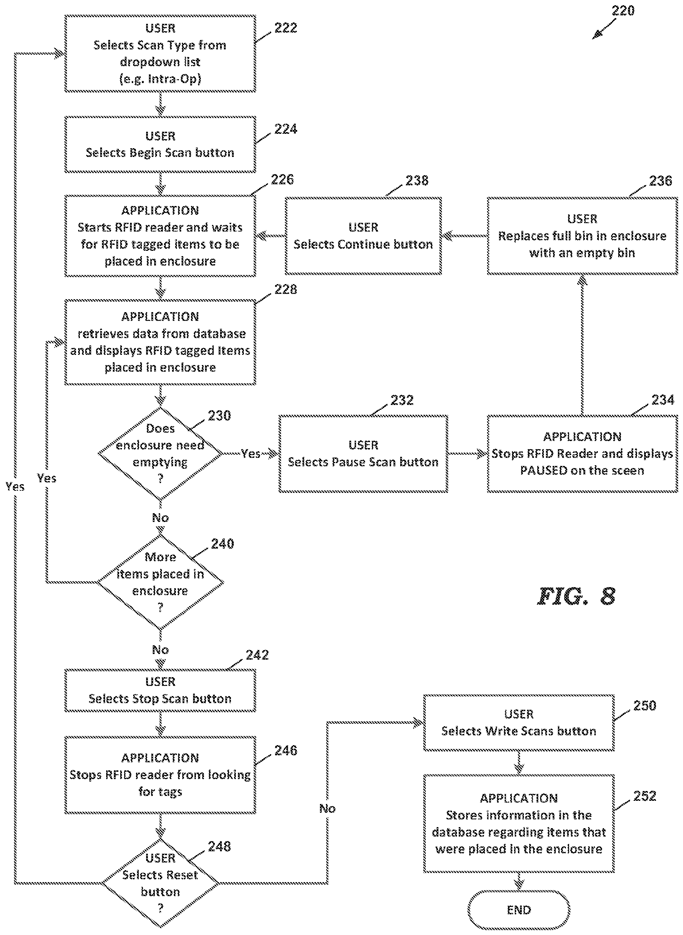

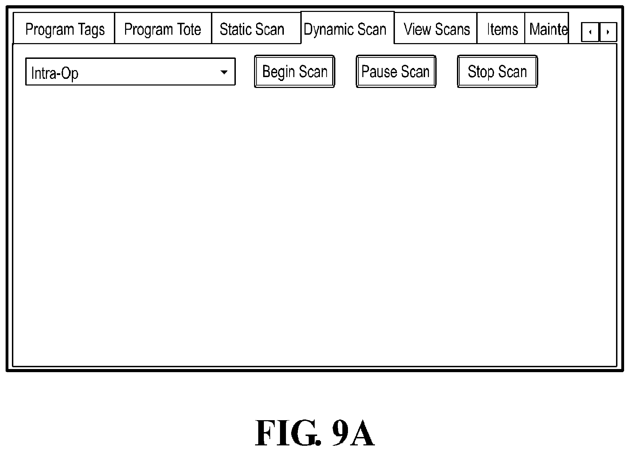

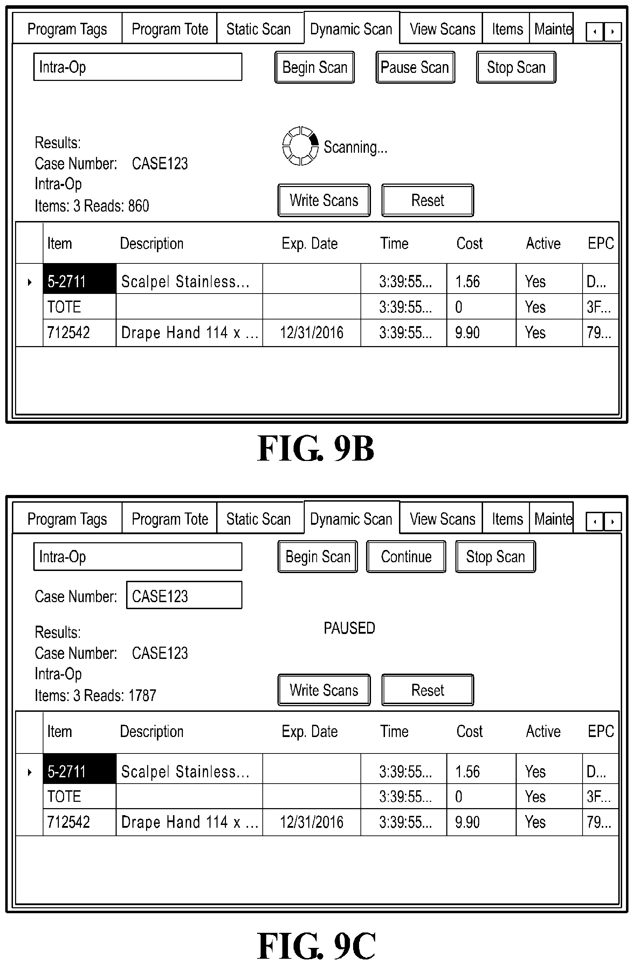

FIG. 8 depicts an example of a method 220 for reading RFID tags on items dropped into the shielded enclosure 12. While running the medical item inventory application, the user selects the "Dynamic Scan" tab on the example display screen depicted in FIG. 9A and selects the scan type, such as "Intra-Op" from the dropdown list (step 222). When the user selects the "Begin Scan" button (step 224), the RFID tag reader 28 is activated and begins reading the tags of any items or item wrappers dropped into the enclosure 12 (step 226). As shown in FIG. 9B, information regarding all tagged items detected by the RFID tag reader is displayed on the display device (step 228). In this example, three tagged items or item wrappers were detected: (1) item 5-2711 Scalpel Stainless..., (2) item TOTE, and (3) item 712542 Drape Hand 114 x.... If at some point during the medical procedure the waste bin within the enclosure needs to be emptied, the user selects the "Pause Scan" button in FIG. 9B (step 232), which causes the application to stop the RFID tag reader and display "Paused" on the screen as shown in FIG. 9C (step 234). After the full bin has been replaced with an empty bin in the enclosure (step 236), the user selects the "Continue" button (step 238), which causes the RFID tag reader 28 to resume reading the tags of any additional items or item wrappers dropped into the enclosure 12 (step 226). When the medical procedure is complete and no more wrappers are to be dropped into the enclosure 12 (step 240), the user selects the "Stop Scan" button (step 242), which causes the RFID tag reader 28 to cease detecting RFID tags in the enclosure (step 246). The user then selects the "Write Scans" button (step 250) at which point the application stores in the database 52 all the item information regarding items or item wrappers that were placed into the enclosure during the medical procedure (step 252).

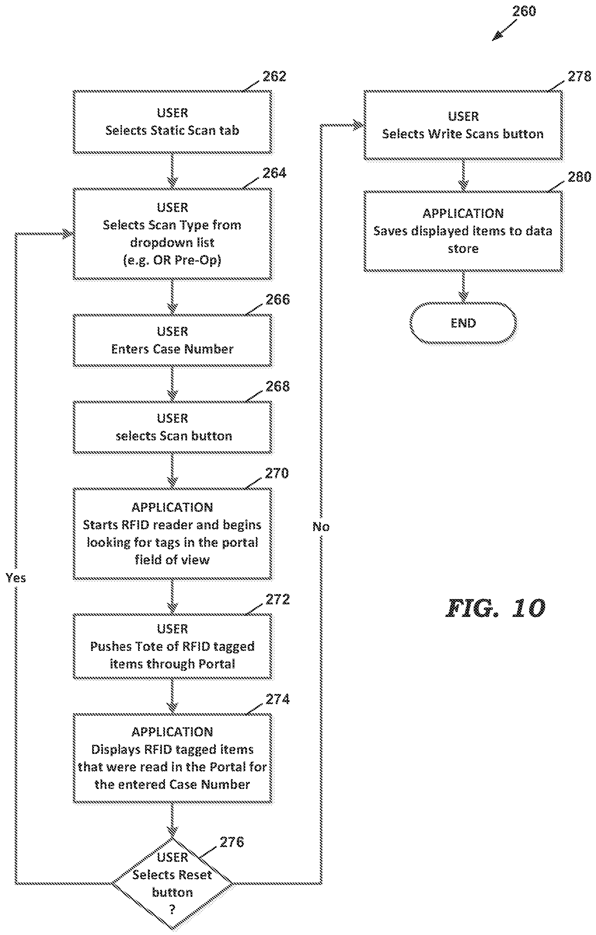

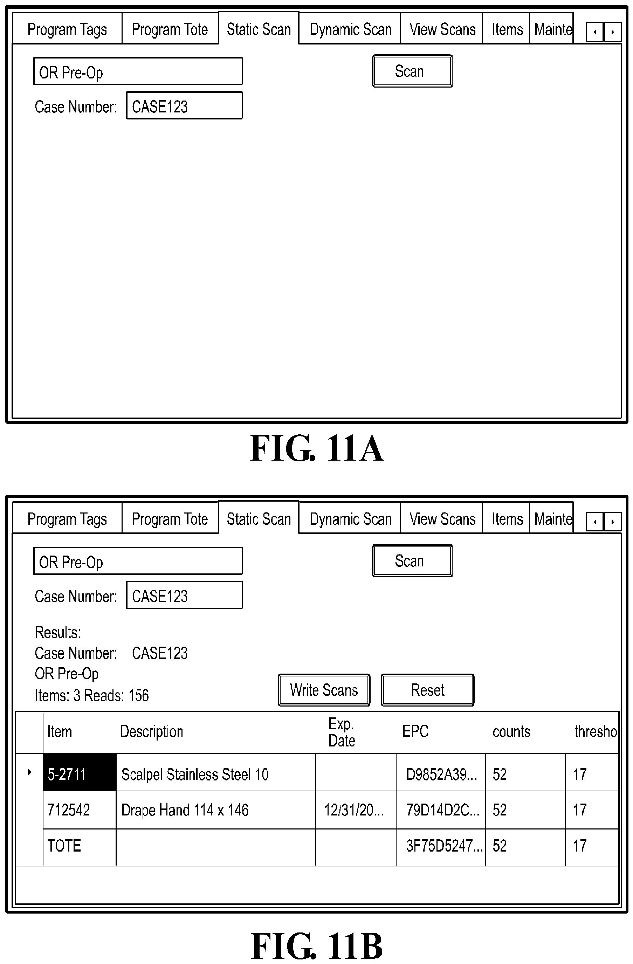

FIG. 10 depicts an example of a method 260 for reading RFID tags on items passed through the portal 48. While running the medical item inventory application, the user selects the "Static Scan" tab (step 262) on the example display screen depicted in FIG. 11A and selects the scan type, such as "OR Pre-Op" from the dropdown list (step 264). The user then enters the case number for the medical procedure (step 266) and selects the "Scan" button (step 268). The application then activates the RFID tag reader 28, which begins reading the tags of any items or item wrappers within the field of view the antennas in the portal opening 49 (step 270). When the user pushes a tote containing RFID-tagged items through the portal opening 49 (step 272), the RFID tag reader 46 reads the tags of the items in the tote and the application displays a list of the items on the display device as shown in FIG. 11B (step 274). The user then selects the "Write Scans" button (step 278) at which point the application stores in the database 52 all the item information regarding items that were passed through the portal (step 280).

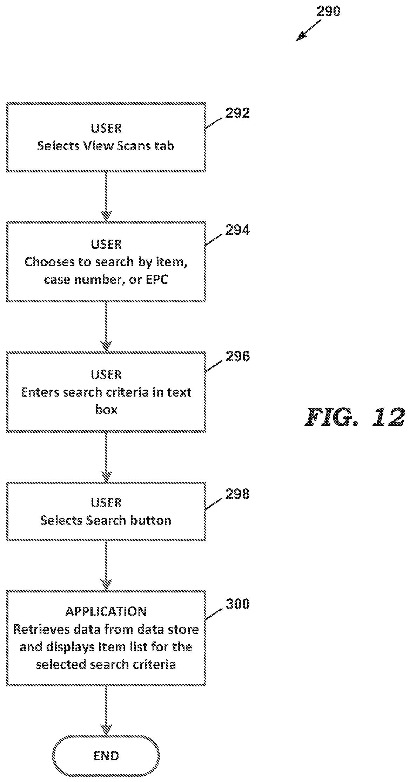

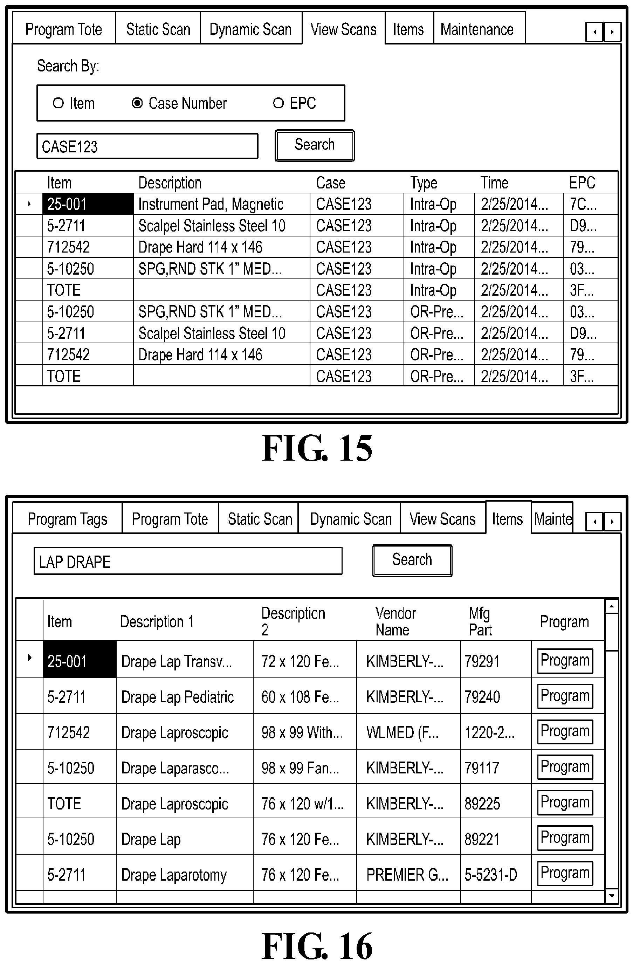

FIG. 12 depicts an example of a method 290 for viewing listings of items whose RFID tags have been read and entered into the database 52. While running the medical item inventory application, the user selects the "View Scans" tab (step 292) on the example display screen as depicted in FIG. 15 and chooses to search by item, by case number or by Electronic Product Code (EPC) (step 294). As will be appreciated by one skilled in the art, the EPC is a unique number that identifies a specific item in the supply chain. When the user enters the search criteria (such as CASE123) in the text box (step 296) and selects the "Search" button (step 298), the application retrieves item information from the database 52 regarding all items scanned in association with CASE123 and displays a list of the item information on the display device as shown in FIG. 15 (step 300).

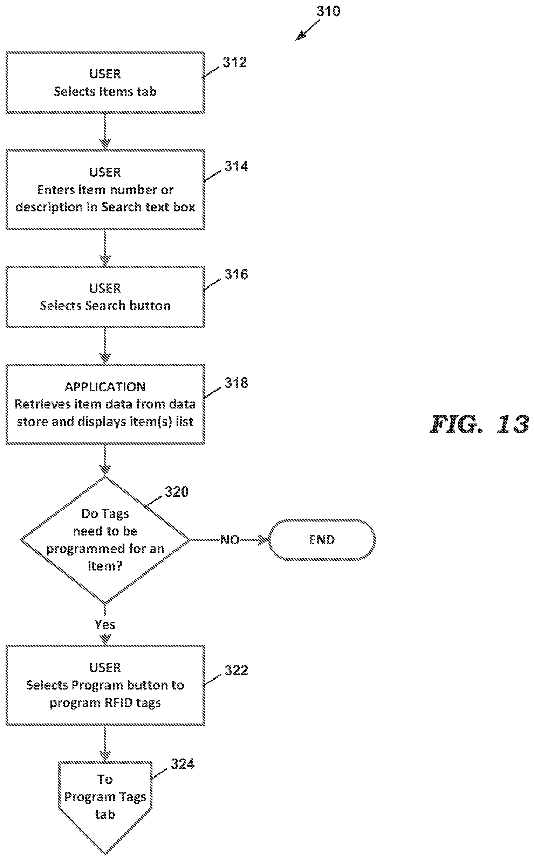

FIG. 13 depicts an example of a method 310 for viewing listings of items having information stored the database 52. While running the medical item inventory application, the user selects the "Items" tab (step 312) on the example display screen as depicted in FIG. 16 and enters an item number or item keywords in the search text box (step 314). When the user selects the "Search" button (step 316), the application retrieves item information regarding all items in the database 52 and displays a list of the item information on the display device as shown in FIG. 16 (step 318). If the list indicates that RFID tags have not yet been programmed tier an item (step 320), the user may select the "Program" button (step 322) which will cause the application to display the "Program Tags" tab (step 324).

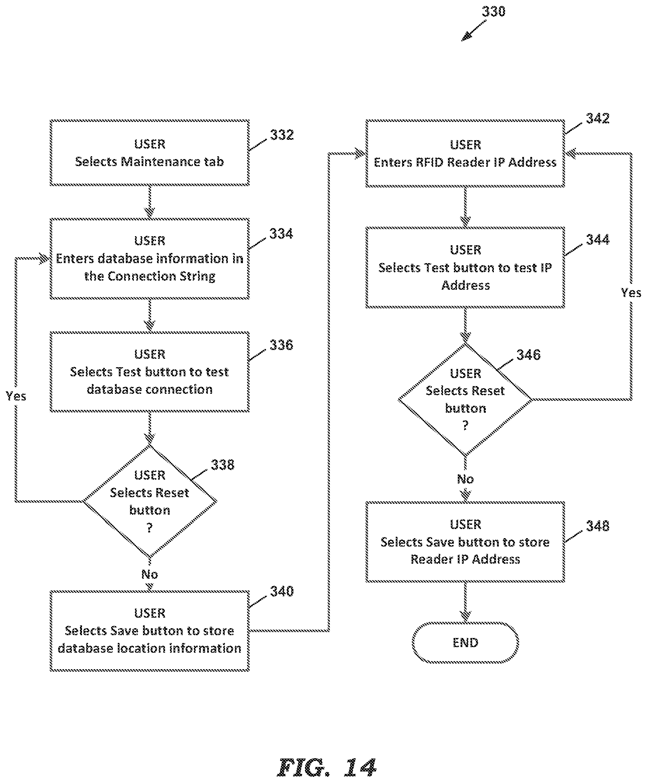

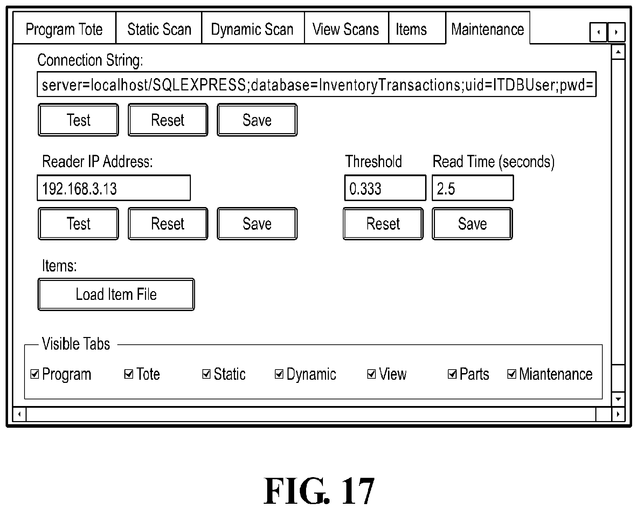

FIG. 14 depicts an example of a method 330 for performing maintenance tasks related to the database 52 and the LAN 42. While running the medical item inventory application, the user selects the "Maintenance" tab (step 332) on the example display screen as depicted in FIG. 17 and enters the network address of the database 52 (step 334). The user may then select the "rest" button to test the connection to the database 52 (step 336). If the test indicates a successful connection, the user may select the "Save" button to store the database address information (step 340). The "Maintenance" tab also allows the user to test the network connection to the RFID tag reader(s) by entering the IP address in the address box (step 342) and selecting the "Test" button (step 344). If the test indicates a successful connection, the user may select the "Save" button to store the -IP address information (step 348).

### Tracking Utilization of Medical Resources in Medical Facility

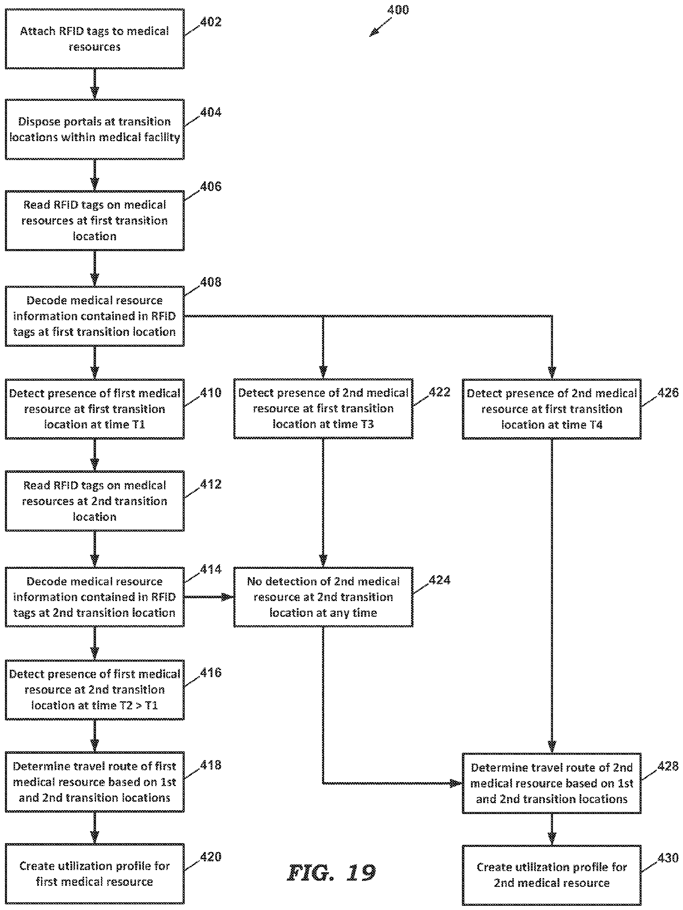

Various embodiments and examples described herein provide systems for sensing RFID tags attached to various medical resources at various transition locations throughout a medical facility, for tracking routes of movement of the medical resources based on the sensing of the RFID tags, for detecting relationships between medical resources based on sensing their RFID tags at the same transition locations during overlapping time periods, for analyzing utilization of the medical resources, and for developing utilization profiles. For example, FIG. 19 depicts an example of a process 400 for analyzing the utilization of two different medical resources based on sensing (or not sensing) their RFID tags at two different transition locations within a medical facility. The process 400 involves attaching RFID tags to medical resources (step 402), disposing RFID-sensing portals at various transition locations within the medical facility (step 404), reading medical resource information from the RFID tags using the portals (step 406 and 412), and decoding the medical resource information to identify the medical resources (step 408 and 414) and determine various characteristics of the resources as described in more detail below.

For example, with continued reference to FIG. 19, a first medical resource is detected at a first transition location at a time T1 (step 410) and at a second transition location at a time T2 (step 416). Based on these detections, the system determines that the first medical resource travelled from the first transition location to the second transition location between times T1 and T2 (step 418). Based on this route of travel and the times of detection, the system creates a utilization profile for the first medical resource (step 420).

A second medical resource is detected at the first transition location at a time T3 (step 422), which may be less than, greater than, or equal to time T1. The second medical resource is again detected at the first transition location at a time T4 (step 426), which is occurs after time T3 (T4 > T3). There is no detection of the second medical resource at the second transition location between times T3 and T4 (step 424). Based on these detections, the system determines that the second medical resource travelled from the first transition location back to the first transition location between times T3 and T4, and did not travel to the second transition location (step 428). Based on this route of travel and the times of detection, the system creates a utilization profile for the second medical resource (step 430).

In the example of FIG. 19, the first transition location may be an entrance/exit door of a medical procedure room PR1 within a medical facility, the second transition location may be a waste container WC1 within the medical procedure room PR1, the first medical resource may be a first medical item that was picked to be used during a medical procedure MP1 in the procedure room PR1, and the second medical resource may be a second medical item that was picked to be used during the same medical procedure MP1 in the procedure room PR1. Based on the detections described above, the system determines that the first medical item entered the medical procedure room PR1 (first transition location) at time T1, and it or its wrapper was deposited in the waste container WC1 (second transition location) at time T2. Based on this route of travel, the system creates a utilization profile indicating that the first medical item was used or consumed during the medical procedure MP1. Also based on the detections described above, the system determines that the second medical item entered the medical procedure room PR1 (first transition location) at time T3, exited the medical procedure room PR1 (first transition location) at time T4, and was not deposited in the waste container WC1 (second transition location). Based on this route of travel, the system creates a utilization profile indicating that the second medical item was brought into the medical procedure room. PR1, but was not used during the medical procedure MP1.

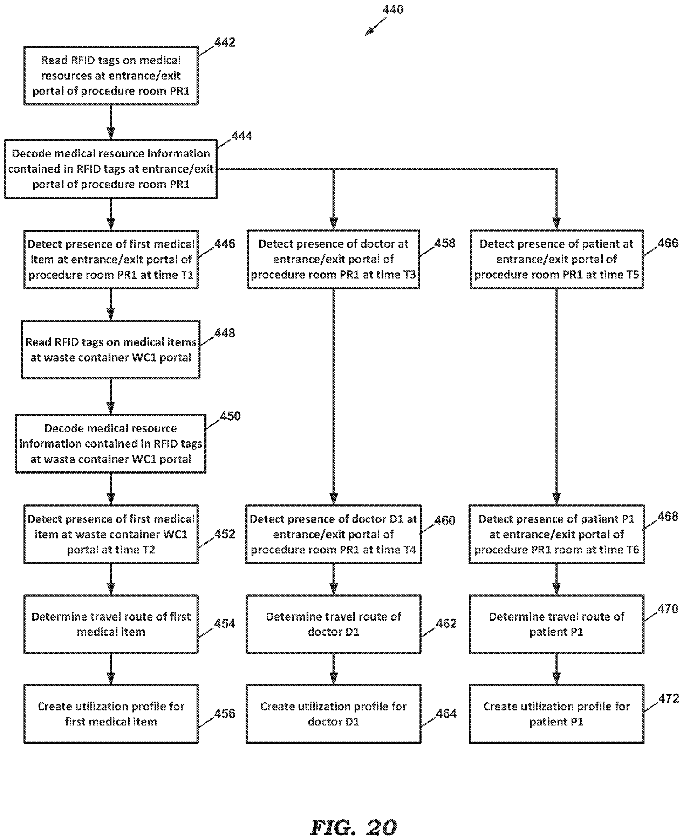

FIG. 20 depicts an example of a process 440 for analyzing the utilization of three different medical resources based on their RFID tags being sensed (or not sensed) at two different transition locations within a medical facility. The process 440 involves reading medical resource information from RFID tags attached to three medical resources - a first medical item, a doctor, and a patient - using portals at the entrance/exit of a procedure room PR1 and on a waste container WC1 (step 442 and 448), and decoding the medical resource information to identify the medical resources (step 444 and 450) and determine various characteristics of the resources. As in the previous example, the system determines that the first medical item entered the medical procedure room PR1 at time T1, and it or its wrapper was deposited in the waste container WC1 at time T2 (step 454). Based on this route of travel, the system creates a utilization profile indicating that the first medical item was used during the medical procedure MP1 (step 456).

With continued reference to FIG. 20, the system detects the doctor D1 entering the medical procedure room PR1 at time T3 which may be less than, greater than, or equal to time T1 (step 458). The doctor D1 is detected leaving the medical procedure room PR1 at time T4 which is greater than T1 and T3 (step 460). Based on this route of travel, the system creates a utilization profile indicating that the doctor D1 was involved in a medical procedure MP1 in the procedure room PR1 between times T3 and T4 (step 464). In examples, the utilization profile for the doctor D1 indicates that the first medical item was consumed or used during a medical procedure MP1 performed by the doctor D1. In some examples, the utilization profile for the first medical item also indicates that the first medical item was consumed or used during a medical procedure MP1 performed by the particular doctor D1.

With continued reference to FIG. 20, the system detects the patient P1 entering the medical procedure room PR1 at time T5 which may be less than, greater than, or equal to time T1 (step 466). The patient P1 is detected leaving the medical procedure room PR1 at time T6 that is greater than T1 and T5 (step 468). Based on this route of travel, the system creates a utilization profile indicating that the patient P1 was involved in a medical procedure MP1 in the procedure room PR1 between times T5 and T6 (step 470). In examples, the utilization profile for the patient P1 also indicates that the first medical item was consumed or used during the medical procedure MP1 performed on the patient P1 by the particular doctor D1. In some examples, the utilization profile for the first medical item also indicates that the first medical item was consumed or used during the medical procedure MP1 performed on the particular patient P1. In some examples, the utilization profile for the doctor D1 also indicates that the first medical item was consumed or used during the medical procedure MP1 performed on the particular patient P1.

### Generating Alerts Based on Utilization of Medical Resources in Medical Facility

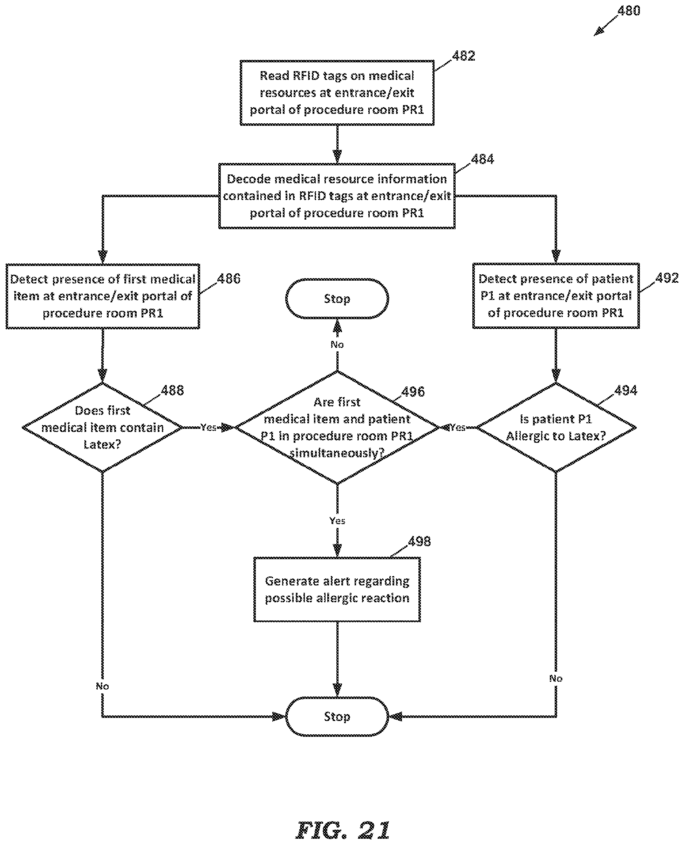

FIG. 21 depicts an example of a process 480 for generating an alert based on utilization of medical resources in the performance of a medical procedure in a medical facility. This process 480 analyzes the utilization of two different medical resources based on sensing their RFID tags at the same transition location within the medical facility. The process 480 involves reading medical resource information from RFID tags attached to the two medical resources - a first medical item and a patient P1 - using portals at the entrance/exit of a procedure room PR1 (step 482), and decoding the medical resource information to identify the medical resources (step 484) and to determine various characteristics of the resources. For example, the medical resource information decoded at step 484 may indicate whether the first medical item contains a potential allergenic, such as Latex, and whether the patient P1 is allergic to any drugs or substances, such as Latex. Using the decoded information, the system detects that the first medical item entered the medical procedure room PR1 (step 486) at a certain time and that the patient P1 entered the medical procedure room PR1 at a certain time (step 492). If the first medical item contains a substance to which the patient P1 is allergic, and the first medical item and the patient P1 are in the procedure room PR1 simultaneously (steps 488, 494 and 496), the system generates an alert informing personnel in the procedure room PR1 of the potential for a harmful allergic reaction (step 498). This alert may be audible (siren) and visible (strobe lights) in the procedure room, and it may be sent via electronic messaging to other personnel within the medical facility to give notice of the situation. In examples, the occurrence of such an event is also reflected in the utilization profile of the patient P1.

In some examples, the system generates a potential allergic reaction alert if an RFID reader portal at the doorway of a supply room detects a medical item leaving the supply room that was picked for use during a medical procedure involving a patient that is allergic to a substance in the medical item. This detection could also be made by any RFID reader portal at any transition location between the supply room and the medical procedure room.

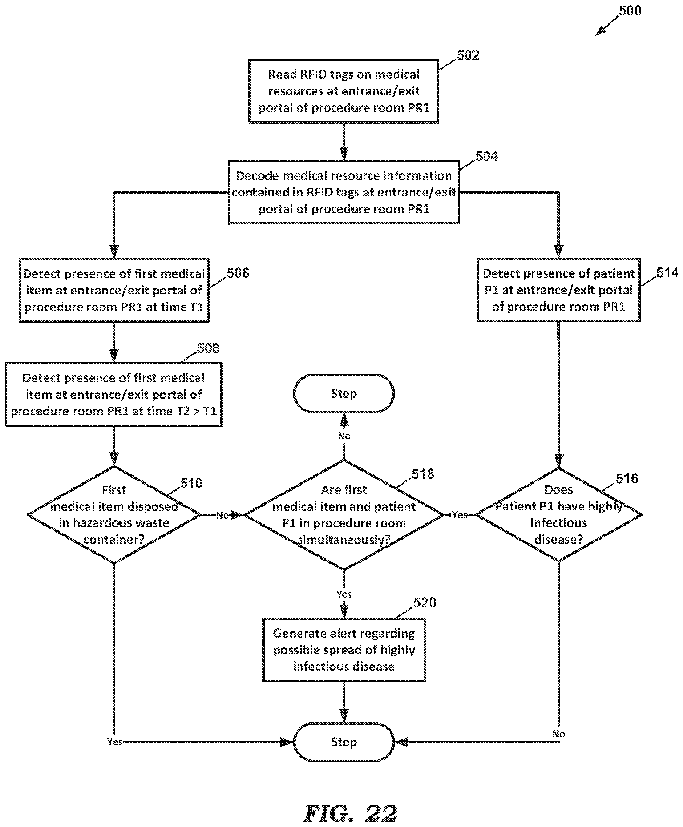

FIG. 22 depicts an example of another process 500 for generating an alert based on utilization of medical resources in the performance of a medical procedure in a medical facility. This process 500 analyzes the utilization of two different medical resources based on sensing their RFID tags at the same transition location within the medical facility. The process 500 involves reading medical resource information from MID tags attached to the two medical resources - a first medical item and a patient P1 - using portals at the entrance/exit of a procedure room PR1 (step 502), and decoding the medical resource information to identify the medical resources (step 504) and to determine various characteristics of the resources. For example, the medical resource information decoded at step 504 may indicate that the patient P1 is infected with a highly infectious contagion, such as Methicillin-resistant Staphylococcus aureus (MRSA). Using the decoded information, the system detects that the first medical item entered the medical procedure room M1 (step 506) at time T1 and that the patient P1 entered the medical procedure room PR1 at a certain time (step 514). The system later detects that the first medical item has exited the medical procedure room PR1 (step 508) at time T2. If the first medical item was not deposited in a hazardous waste container prior to leaving the procedure room PR1, and the first medical item and the patient P1 were in the procedure room PR1 simultaneously, and the patient P1 is infected with a contagion such as MRSA (steps 510, 516, 518), the system generates an alert informing personnel in the procedure room PR1 of a potential for spread of a highly infectious contagion due to possible contact with the first medical item (step 520). This alert may be audible (siren) and visible (strobe lights) in the procedure room, and it may be sent via electronic messaging to other personnel within the medical facility to give notice of the situation. In examples, the occurrence of such an event is also reflected in the utilization profile of the first medical item. In some situations, the determination that the patient is infected (step 516) may be made after the procedure is complete and the patient has left the procedure room. In such situations, the system will generate the alert (step 520) after information indicating the patient's infection is entered into the patient's record (the medical resource information for the patient.)

### Tracking Custody of Medical Items in Supply and Consumption Chain

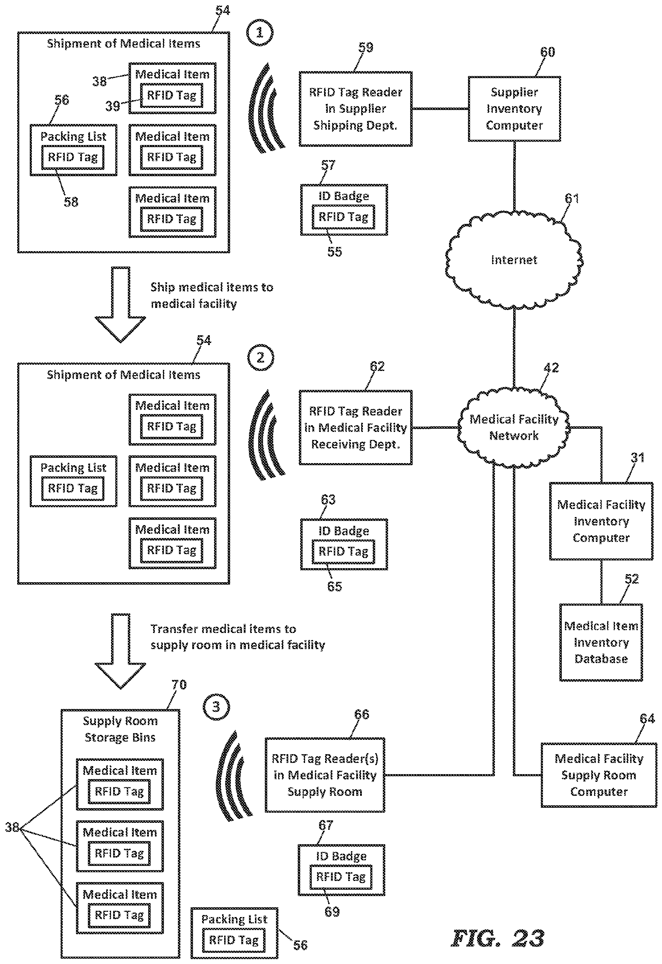

FIGS. 23-25 depict a system for tracking medical items through various transition points in a supply and consumption chain. Unless otherwise defined herein, the term "supplier" generally refers to a medical item manufacturer or distributor or any other entity that ships medical items to a medical facility to be put into inventory. As shown in FIG. 23, each shipment 54 of medical items 38 from a supplier typically includes a packing list 56 that has a unique shipment identifier 58 encoded in an RFID tag and/or a bar code attached to the packing list or attached to a container in which the medical items 38 are shipped. Upon packing of medical items 38 at a supplier facility for shipment (first transition point), a unique item identifier 39 encoded in an RFID tag and/or a bar code attached to each packed medical item 38 and the unique shipment identifier 58 are read by one or more reading devices 59 at the supplier facility and are cross-referenced in a supplier inventory database 60. In examples, the reading devices 59 may include one or more RFID tag readers and their associated antennas and one or more bar code readers. The one or more reading devices 59 may also read the unique personnel identifier 55 of the person responsible for the shipment of medical items at the supplier facility, which identifier may be encoded in the person's ID badge 57. In an example, the unique shipment identifier 58 is also cross-referenced in the supplier inventory database 60 with the unique personnel identifier 55. As the term is used herein, a "supplier facility" is any location at which the medical items are disposed prior to shipment to the medical facility, such as a manufacturer facility or a distributor facility.

Upon receipt of the shipment 54 of medical items at the medical facility (second transition point), the unique shipment identifier 58 of the packing list 56 is read and decoded by one or more reading devices 62, and the unique shipment identifier is stored in the medical facility inventory database 52 in association with a unique personnel identifier 65 of the person responsible for receiving the shipment of medical items at the medical facility. In examples, the one or more reading devices 62 may include one or more RFID tag readers and their associated antennas and one or more bar code readers. The one or more reading devices 62 may also read the unique personnel identifier 65 of the person responsible for receiving the shipment of medical items at the medical facility, which identifier may be encoded in the person's ID badge 63.

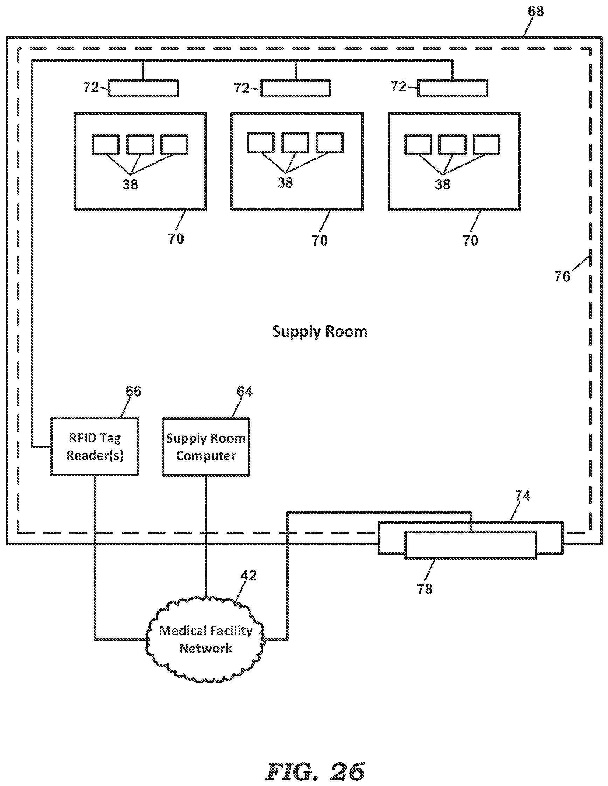

Upon unpacking a received shipment 54 of medical items and placement into storage bins 70 in a supply room 68 at the medical facility (third transition point), the unique shipment identifier 58 and the unique item identifier 39 on each medical item 38 is read and decoded by one or more reading devices 66 in the supply room 68, and the unique item identifier 39 of each medical item 38 is stored in association with a unique supply room identifier in the inventory database 52 for the medical facility. The supply room 68 may also be referred to herein as a "vault" and it may be electromagnetically shielded as depicted in FIG. 26 and described in more detail hereinafter. In preferred examples, the one or more reading devices 66 in the supply room 68 may include one or more RFID tag readers and their associated antennas and one or more bar code readers. In examples, the inventory database 52 also cross-references the unique item identifiers 39 with a unique personnel identifier 69 of the person responsible for placement of the medical items into inventory at the medical facility, which identifier may be encoded in the person's ID badge 67.

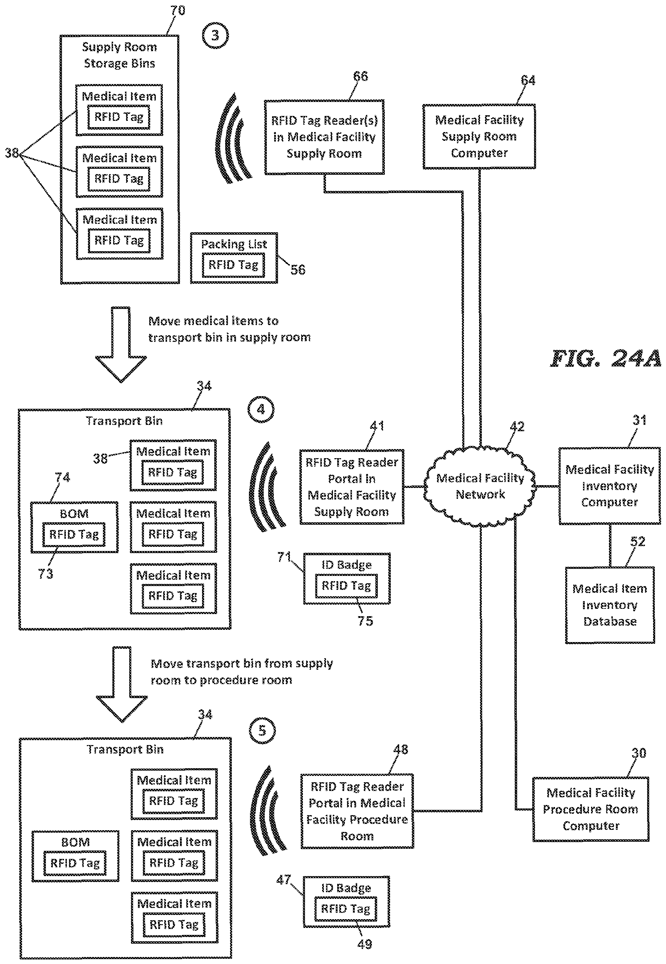

As shown in FIG. 24A, upon pulling medical items 38 from the supply room storage bins 70 to be used in a medical procedure, examination or test, or to be otherwise dispensed to a patient (fourth transition point), the unique item identifier 39 on each medical item 38 is read and decoded by a portal reading device 41 in the supply room, and the unique item identifier of each medical item is stored in the inventory database 52 in association with a unique bill of material (BOM) identifier 73 of the BOM 74 for the procedure/exam/test. In an example, the unique item identifiers 39 and BOM identifier 73 are cross-referenced in the database 52 with a unique personnel identifier 75 of the person removing the medical items from the supply room, which identifier may be encoded in the person's ID badge 71. At this point, the medical items may be in a transport bin 34 or other container 34.

Upon delivery of the medical items 38 to a procedure room, examination room, or test room in the medical facility (fifth transition point), the unique item identifier 39 on each medical item is read and decoded by a portal reading device 48 in or at the entrance to the procedure/exam/test room, and the unique item identifier of each medical item is stored in the inventory database 52 in association with a unique procedure/exam/test room identifier and a unique personnel identifier 49 of a person accepting delivery in the procedure/exam/test room. The unique personnel identifier 49 may be encoded in the person's ID badge 47.

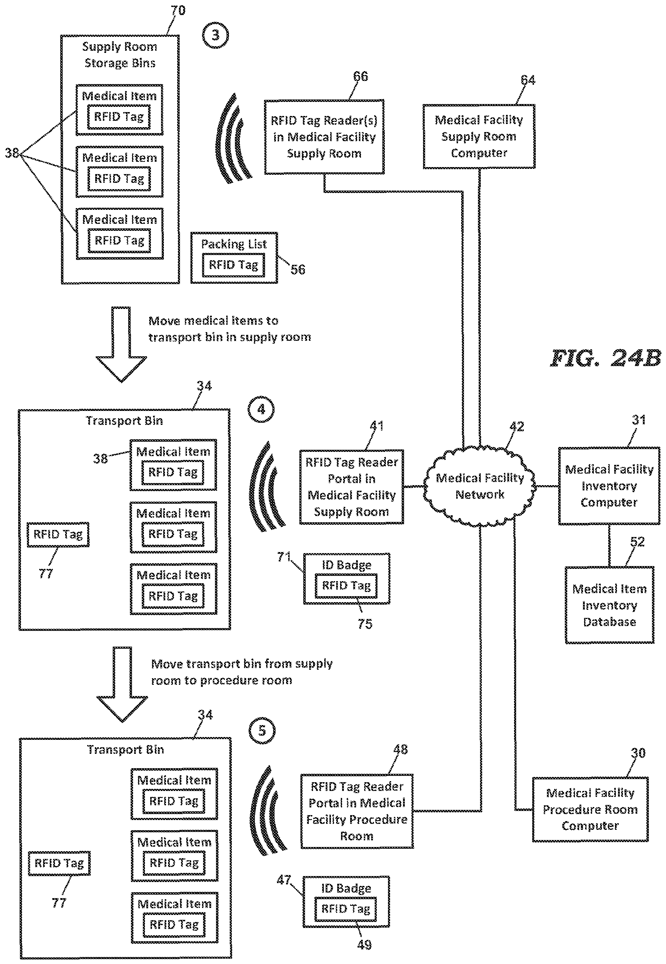

In an alternative example depicted in FIG. 24B, upon pulling the medical items 38 from the supply room storage bins 70 to be used in a medical procedure, examination or test, or to be otherwise dispensed to a patient (fourth transition point), the unique item identifier 39 on each medical item 38 is read and decoded by a portal reading device 41 in the supply room, and the unique item identifier of each medical item is stored in the inventory database 52 in association with a transport bin identifier 77 of the transport bin 34 used to transport the items to a procedure/exam/test room. The unique item identifiers 39 and transport bin identifier 77 are preferably cross-referenced in the database 52 with the unique personnel identifier 75 of the person removing the medical items from the supply room, which identifier may be encoded in the person's ID badge 71. In this example, the transport bin 34 may contain medical items 38 that are to be used in the treatment of several different patients in the medical facility. In this case, the unique item identifiers 39 will become associated with a particular patient only after the medical items 38 are delivered to a particular procedure/exam/test room in which the patient is being treated.

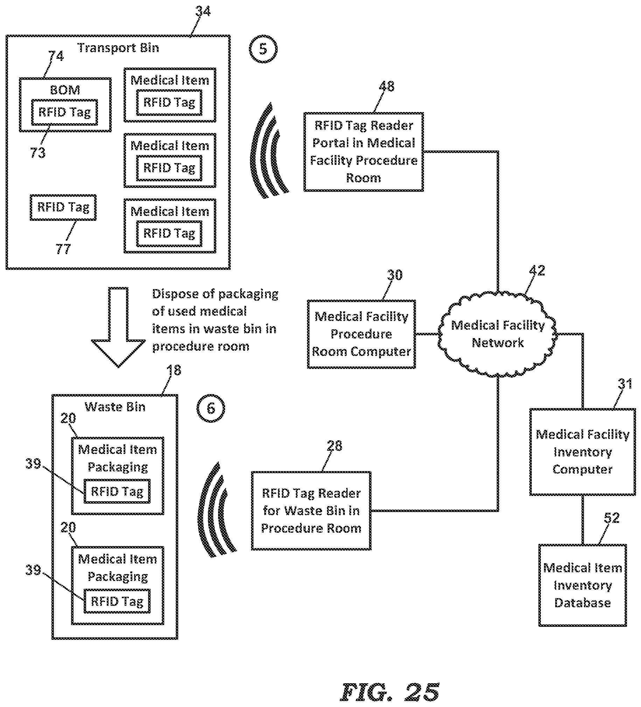

As shown in FIG. 25, upon dispensing the medical items 38 to a patient or otherwise consuming the medical items during a procedure or test performed on the patient in the procedure/exam/test room (sixth transition point), the unique item identifier 39 on the packaging 20 of each used medical item is read and decoded by a reading device 28 associated with the waste bin 18 in the procedure/exam/test room, and the unique item identifier of each medical item is stored in the inventory database 52 in association with a unique patient identifier.

The medical items 38 that are delivered to the procedure/exam/test room but not used or consumed during the medical procedure are preferably passed through the portal 48, and the reader 46 reads the RFID tags of the unused items 38. In an example, the item identification information encoded in those RFID tags is cross-referenced in the database 52 with a unique personnel identifier of the person responsible for removing the unused medical items from the medical procedure room and transporting the items to the supply room for restocking, which identifier may be encoded in the person's ID badge.

Medical items 38 in the transport bin 34 that are not delivered to a procedure/exam/test room preferably remain associated in the database 52 with the identifier of the person who originally removed the items from the supply room. When the unused items have been returned to the supply room and their RFID tags are read by the RFID reader 41 in the supply room, their identifiers automatically become disassociated with the person who removed the items from the supply room. Those items also are once again recorded in the database 52 as being present in the supply room.

At each transition point, a prompt may be automatically generated to remind the responsible personnel to scan the unique item identifier 39 on each medical item 38 so that the information will be entered into the inventory database 52, or to remind the responsible personnel to take other action as may be necessary based on the location and status of the medical items 38. These prompts may be visual or audible.

In this example, the inventory database 52 maintains a chain of custody for each item 38 through each transition point (and for each shipment 54 of items between the first and second transition points) and keeps records of the personnel responsible for each item or shipment at each location at any particular time.

At some transition points, individual medical item information may not be specifically recorded, although bulk information associated with the packing list 56 will be recorded. When the medical items are dispensed to the subsequent transition point, the unique item identifiers 39 may recovered and the unique item identifier information can be automatically associated with the dispensing activity where unique item identifier information was previously not recorded.

Although a particular sequence of transition points is described, transition points could consist of any handling point along the supply chain for a medical item from manufacturer to patient.

In further examples, each transition point is defined by the type of location. Each type of location may have a set of characteristics associated with that location type that trigger certain action prompts when the medical items are associated with that particular location type in the database 52. For example, when the shipment 54 of medical items is received at a supply room 68, such as by scanning the identification information on a packing list 56, the supply room identification equipment may prompt the inventory management personnel to place the products in the appropriate product storage location, initiate an RFID scan of the room to identify the medical items present in the room and subsequently present information to the inventory management personnel to determine if the room inventory, as updated, reconciles with the packing list.

In another example, when the RFID reader 48 in a procedure/examination room detects in the room a medical item that is considered to be Durable Medical Equipment, Prosthetics, Orthotics and Supplies (DMEPOS, also collectively referred to herein simply as "DME"), and it also detects a patient identification number encoded in an RFID tag associated with a particular patient in the room, the procedure room computer 30 may generate action prompts based on association of the DME item with the patent. For example, the procedure room computer 30 may prompt the treating personnel to disclose to the patient certain information related to the proper use of the DME item, to input information to verify the delivery of the DME item to the patient, or to obtain the patient's signature to acknowledge receipt of the DME item. In some examples, custody of a DME item passes from the treating personnel to the patient only after the patient has entered a digital signature on a tablet computer or other similar interface device. In a situation in which an old or used DME item is detected in the room, and the patient is supposed to receive a new item, the system may generate a notification to treating personnel that the old/used item should go back to hospital stock and a new item should be dispensed to patient.

In some examples, the exits of the hospital or other medical facility are transition points at which networked RFID tag readers are positioned. When a just-dispensed DME item is detected at any of these exit transition points, the medical item inventory computer 31 triggers a billing change event to cause the billing for the DME item to change from Medicare Part A, in which the medical facility pays for rental, to Medicare Part B, in which the patient or the patient's insurance company is billed for the item. This exit event may also cause the medical item inventory computer 31 to update the chain of custody for the DME item to indicate a transfer of possession from the medical facility to the patient. Such an exit event may also trigger the sending of notifications to the patient and medical personnel regarding follow-up care for patient using the DME item, such as notifications recommending a Part B healthcare provider and prompting the scheduling of follow-up appointments.

### Medical Item Supply Room

FIG. 26 depicts a functional block diagram of features of a medical item supply room 68 according to an example. Within the supply room 68 are one or more storage bins 70 in which medical items 38 are stored until they are needed for treatment of a patient. As the term is used herein, a "storage bin" is any storage structure in or on which medical items may be stored, including but not limited to a container, shelf, drawer, hanger, or cabinet. One or more RFID antennas 72 are directed toward the storage bins 70 to detect RFID tags on medical items 38 stored therein. The antennas 72 are electrically connected to one or more RFID tag readers 66 that are in communication with the medical facility network 42. In some examples, a supply room computer 64 is provided to allow personnel in the supply room 68 to access medical item inventory information over the network 64.

In some examples, the supply room 68 has RF shielding 76 in the wails, floor and ceiling to prevent detection of RFID tags that are outside the supply room. RFID shielding 76 may also be provided in gaskets around the edges of the door frame and in a door sweep on the bottom of the door.

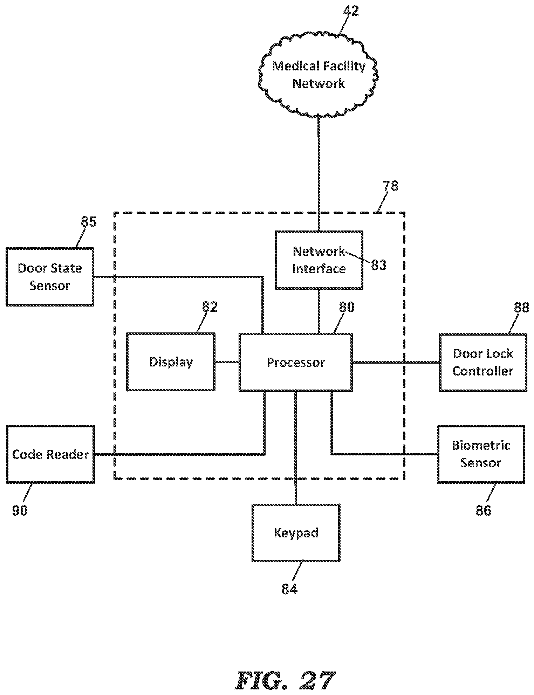

In some examples, a computer 78 is built into the door 74 of the medical item supply room 68. As shown in FIG. 27, this computer 78 preferably includes a processor 80, a touch screen display 82, keypad 84, biometric sensor 86 such as a fingerprint reader or retinal scanner, and a code reader 90, such as an RFID reader, barcode reader or magnetic stripe reader, for reading identification information of personnel seeking access to the supply room and decoding the unique personnel identifiers encoded personnel ID badges. In an example, the processor 80 interfaces with one or more sensors 85 that sense whether the door 74 is in an open state or a closed state and/or whether the door is locked or unlocked.

The computer 78 may also interface with a door lock controller 88 to allow or deny access to the room based on the identity of the person seeking access and based on ongoing activities in the room. For example, if another person is in the room entering items into inventory, the computer may be programmed to not allow access so as not allow outside RFID signals into the room and interfere with the ongoing inventory activity. The computer 78 may also be programmed to keep the door locked while an RFID scan is taking place, whether or not anyone is in the room. The computer 78 may also be programmed to deactivate the RFID reader 66 inside the room when the door is open so that no RFID tags on items outside the room will be detected.

In some examples, the supply room computer 64 or the computer 78 controls the RFID tag reader(s) 66 to perform an RFID antenna calibration procedure. This procedure may involve placing a known number of RFID-tagged items in various different storage bins 70 in the supply room 68 with the RFID tag reader 66 initially deactivated. The computer 64 then activates the RFID tag reader 66 at a first relatively low transmitter power level and the number of tags detected is noted. If not all the tags present in the room were detected, the transmitter power level is increased by a small amount to a second power level that is greater than the first power level and the number of tags detected is noted. This procedure is repeated until all tags are detected, and the lowest transmitter power level at which all tags were detected is stored as the optimum operational level. This general procedure could also be performed by starting at a relatively high transmitter power level at which all tags are detected and then stepping down in power until not all of the tags are detected.

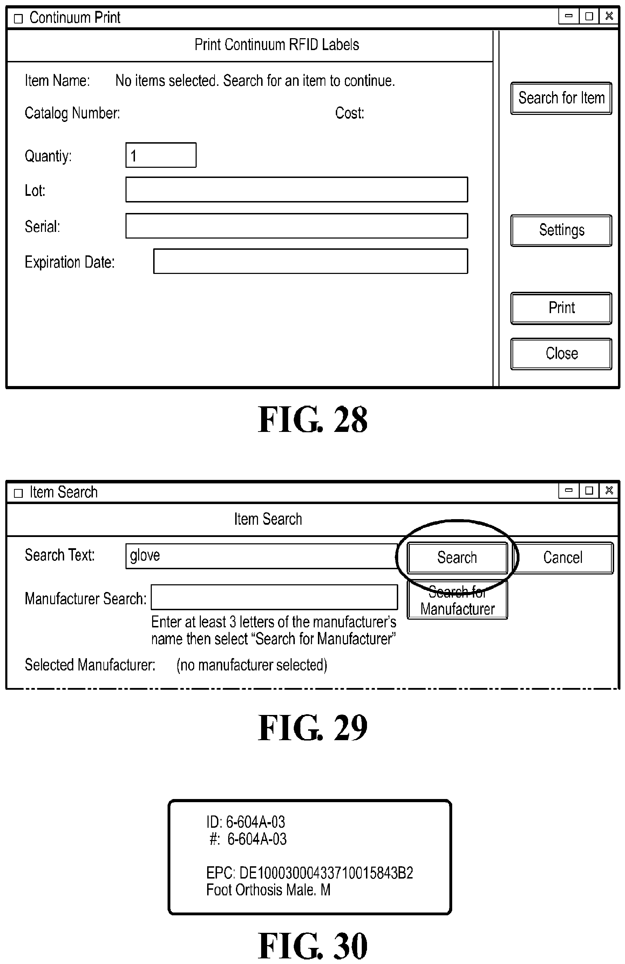

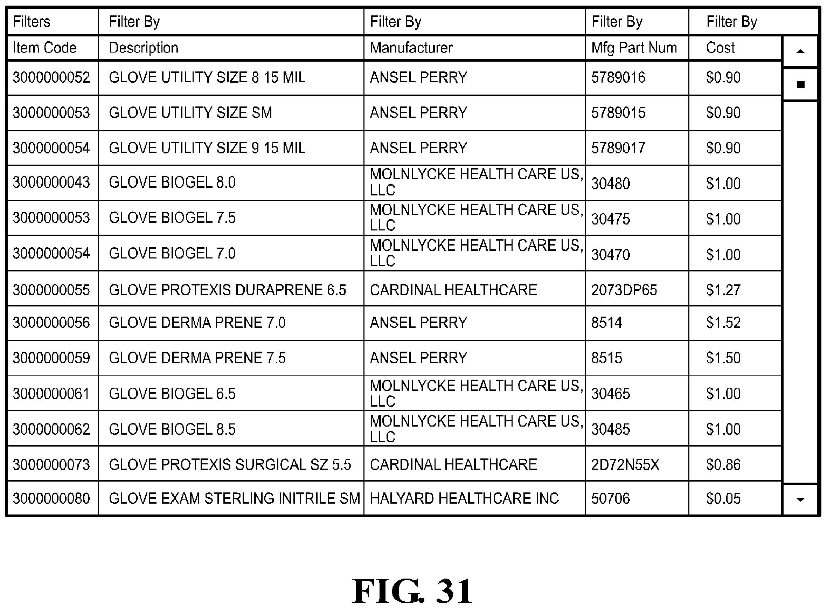

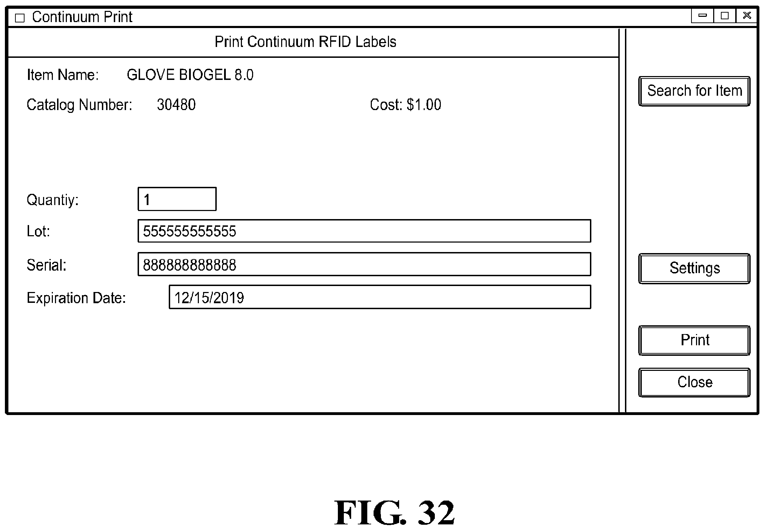

Some examples provide for printing information regarding a medical item on an RFID label and electronically encoding the information in the label. After loading RFID labels into an RFID label printer and calibrating the printer, a print utility application is executed on a user computer that is networked with the RFID label printer. An exemplary user interface screen generated by the printer utility application is depicted in FIG. 28. From this screen, the user clicks on the "Search for Item" button, at which point the printer utility application generates the user interface screen depicted in FIG. 29. The user types in a part description (i.e., "glove") or a manufacturer product code in the "Search Text" field and clicks the "Search" button. Alternatively, the user may type in a manufacturer name in the "Manufficturer Search" field and click the "Search for Manufacturer" button. At this point the printer utility application generates the user interface screen depicted in FIG. 31, which lists all items having "glove" in the item description. The user clicks on one of the listed items to select it, at which point the printer utility application generates the user interface screen depicted in FIG. 32. The user then enters the quantity, lot number, serial number, and expiration date in the corresponding fields depicted in FIG. 32, and clicks on the "Print" button. An example of a printed RFID label is depicted in FIG. 30. The user then inspects the printed RFID label to confirm that the correct information has printed and that it is properly aligned. In an example, if "VOIDVOIDVOID" is printed on the label, the user should repeat the printer calibration steps.

The foregoing description of embodiments for this invention and examples have been presented for purposes of illustration and description. They are not intended to be exhaustive or to limit the invention to the precise form disclosed. Obvious modifications or variations are possible in light of the above teachings. The embodiments and examples are chosen and described in an effort to provide the best illustrations of the principles of the invention and its practical application, and to thereby enable one of ordinary skill in the art to utilize the invention in various embodiments and with various modifications as are suited to the particular use contemplated. All such modifications and variations are within the scope of the invention as determined by the appended claims when interpreted in accordance with the breadth to which they are fairly, legally, and equitably entitled.

## Claims

1. An apparatus for tracking custody of medical items (38) in a supply and consumption chain, wherein each medical item (38) has an RFID tag attached thereto that encodes medical item information specific to the medical item (38), wherein the medical items (38) are initially collected together as a shipment (54) of medical items (38), and wherein the shipment (54) has a packing list (56) associated therewith and the packing list (56) has a shipment identifier (58) associated therewith that encodes shipment identification information specific to the shipment (54), the apparatus comprising:
a first reading device (59) disposed in an area of a medical facility at which the shipment (54) of the medical items (38) is received, the first reading device (59) for reading the shipment identifier (58) associated with the packing list (56) and decoding the shipment identification information encoded in the shipment identifier (58);
one or more first RFID antennas (72) disposed in a supply room (68) in the medical facility, the one or more first RFID antennas (72) for receiving first radio frequency signals emanated from the RFID tags attached to the medical items (38) in the shipment (54) after the medical items (38) have been brought into the supply room (68), wherein the first radio frequency signals contain the medical item information encoded in the RFID tags attached to the medical items (38);
one or more first RFID readers (66) associated with the supply room (68) of the medical facility and electrically connected to the one or more first RFID antennas (72), the one or more first RFID readers (66) for decoding the medical item information contained in the first radio frequency signals emanated from the RFID tags attached to the medical items (38);
a second reading device (66) associated with the supply room (68) of the medical facility, the second reading device (66) for reading at least the shipment identifier (58) associated with the packing list (56) and decoding the shipment identification information encoded in the shipment identifier (58);
a medical facility inventory computer (31) in electrical communication with the first reading device (59); the one or more first RFID readers (66); and the second reading device (66);
a medical item inventory database (52) in electrical communication with the medical facility inventory computer (31);
a shielded enclosure (12) disposed in a medical procedure room (PR1), the shielded enclosure (12) comprising:
an internal space for receiving wrappers (20) of medical items (38) used during a medical procedure (MP1);
an opening (24) in the top of the shielded enclosure (12) through which the wrappers (20) are deposited; and
a cover (25) provided above the opening (24) to prevent RFID signals from escaping the shielded enclosure (12),
the shielded enclosure (12) configured to attenuate radio frequency signals emanated from RFID tags disposed outside the shielded enclosure (12) to levels that are substantially undetectable within the internal space;
a waste bin (18) for receiving wrappers (20) of medical items (38) used during the medical procedure (MP1) disposed within the internal space of the shielded enclosure (12);
one or more fourth RFID antennas (14a, 14b) disposed within the internal space of the shielded enclosure (12), the one or more fourth RFID antennas (14a, 14b) for receiving fourth radio frequency signals emanated from RFID tags attached to the wrappers (20) disposed within the internal space, wherein the fourth radio frequency signals contain the medical item information encoded in the RFID tags; and
at least one fourth RFID reader (28) electrically connected to the one or more fourth RFID antennas (14a, 14b), the at least one fourth RFID reader (28) for decoding the medical item information contained in the fourth radio frequency signals emanated from the RFID tags attached to the wrappers (20) disposed within the internal space,
wherein:
the medical facility inventory computer (31) is programmed to automatically associate a patient identifier with the medical item information contained in the fourth radio frequency signals, wherein the association is triggered by the RFID tags of the medical items (38) in the shipment (54) being detected by the fourth RFID reader (28), and wherein the patient identifier identifies a patient on which the medical procedure (MP1) was performed in the medical procedure room (PR1);
the medical facility inventory computer (31) is further programmed to receive the shipment identification information, the medical item information, a first personnel identifier (65) that identifies a person responsible for receiving the shipment (54) of medical items (38) at the medical facility, a second personnel identifier (69) that identifies a person responsible for placement of the medical items (38) into inventory at the medical facility, and a supply room identifier that identifies the supply room (68) into which the medical items (38) are placed into inventory;
the medical item inventory database (52) is for associating at least the shipment identification information with the first personnel identifier (65), the second personnel identifier (69), and the supply room identifier; and
the medical facility inventory computer (31) is programmed to automatically generate one or both of a first message directed to the person responsible for receiving the shipment (54) of medical items (38) at the medical facility and a second message directed to the person responsible for placement of the medical items (38) into inventory at the medical facility, the generation of the first message triggered by the shipment identification information becoming associated with the first personnel identifier (65), and the second message triggered by the shipment identification information becoming associated with the second personnel identifier (69), wherein the first and second messages prompt the person to whom they are directed to take some action with regard to the shipment (54) of medical items (38).

2. The apparatus of claim 1, wherein:
the shipment identifier (58) comprises an RFID tag attached to the packing list (56), and wherein the first reading device (59) comprises an RFID reader;
the second reading device (66) comprises one of the one or more first RFID readers (66) associated with the supply room (68); and
the medical facility inventory computer (31) is operable to automatically associate the supply room identifier with the medical item information of the medical items (38) in the shipment (54), wherein the association is triggered by the RFID tags on the medical items (38) in the shipment (54) being detected by the one or more first RFID readers (66) or the shipment identifier (58) being read by the second reading device (66).

3. The apparatus of claim 1, wherein the medical item inventory database (52) maintains a chain of custody of the medical items (38) in the shipment (54) based on cross-referencing the shipment identification information with the first personnel identifier (65) and the second personnel identifier (69).

4. The apparatus of claim 1, further comprising:
a supply room portal having:
a portal opening; and
one or more second RFID antennas having fields of view directed to the portal opening, the one or more second RFID antennas for receiving second radio frequency signals emanated from RFID tags attached to medical items (38) that pass through the portal based on the medical items (38) being picked for removal from the supply room (68) to be used in the medical procedure (MP1); and
a second RFID reader electrically connected to the one or more second RFID antennas of the supply room portal, the second RFID reader for decoding the medical item information contained in the second radio frequency signals emanated from the RFID tags, wherein:
the medical facility inventory computer (31) being programmed to automatically associate a third personnel identifier (75) with the medical item information contained in the second radio frequency signals, wherein the association is triggered by the RFID tags on the medical items (38) in the shipment (54) being detected by the second RFID reader, and wherein the third personnel identifier (75) identifies a person responsible for removal of the medical items (38) from the supply room (68).

5. The apparatus of claim 4, wherein the medical item inventory database (52) maintains a chain of custody of the medical items (38) in the shipment (54) based on cross-referencing the medical item information contained in the second radio frequency signals with the third personnel identifier (75).

6. The apparatus of claim 4, wherein the medical facility inventory computer (31) is operable to automatically generate a third message directed to the person responsible for removal of the medical items (38) from the supply room (68), the generation of the third message triggered by the medical item information contained in the second radio frequency signals becoming associated with the third personnel identifier (75), wherein the third message prompts the person to whom it is directed to take some action with regard to the medical items (38) that have been removed or are to be removed from the supply room (68).

7. The apparatus of claim 4, wherein the medical items (38) that have been picked for removal from the supply room (68) to be used in the medical procedure (MP1) are disposed in a transport container (34) that includes an RFID tag (77) attached thereto that encodes transport container identification information,
wherein:
the one or more second RFID antennas of the supply room portal are for receiving a radio frequency signal emanated from the RFID tag (77) attached to the transport container (34) as it passes through the portal;
the second RFID reader is for decoding the transport container identification information contained in the radio frequency signal emanated from the RFID tag (77) attached to the transport container (34); and
the medical facility inventory computer (31) is programmed to automatically associate the transport container identification information with the medical item information of the medical items (38) disposed in the transport container (34).

8. The apparatus of claim 1, further comprising:
a procedure room portal (48) having:
a portal opening (49); and
one or more third RFID antennas (50a-50d) having fields of view directed to the portal opening (49), the one or more third RFID antennas (50a-50d) for receiving third radio frequency signals emanated from RFID tags attached to medical items (38) that pass through the procedure room portal (48) when the medical items (38) are brought into a medical procedure room (PR1) to be used in the medical procedure (MP1); and
a third RFID reader (46) electrically connected to the one or more third RFID antennas (50a-50d) of the procedure room portal (48), the third RFID reader (46) for decoding the medical item information contained in the third radio frequency signals emanated from the RFID tags,
wherein:
the medical facility inventory computer (31) is programmed to automatically associate a fourth personnel identifier with the medical item information contained in the third radio frequency signals, wherein the association is triggered by the RFID tags on the medical items (38) in the shipment (54) being detected by the third RFID reader (46), and wherein the fourth personnel identifier identifies a person responsible for the medical items (38) while the medical items (38) are in the medical procedure room (PR1).

9. The apparatus of claim 8, wherein the medical item inventory database (52) maintains a chain of custody of the medical items (38) in the shipment (54) based on cross-referencing the medical item information contained in the third radio frequency signals with the fourth personnel identifier.

10. The apparatus of claim 8, wherein the medical facility inventory computer (31) is operable to automatically generate a fourth message directed to the person responsible for the medical items (38) while the medical items (38) are in the medical procedure room (PR1), the generation of the fourth message triggered by the medical item information contained in the third radio frequency signals becoming associated with the fourth personnel identifier, wherein the fourth message prompts the person to whom it is directed to take some action with regard to the medical items (38) brought into the medical procedure room (PR1).

11. The apparatus of claim 1, wherein the medical item inventory database (52) maintains a chain of custody of the medical items (38) in the shipment (54) based on cross-referencing the medical item information contained in the fourth radio frequency signals with the patient identifier.

12. The apparatus of claim 1, wherein the medical facility inventory computer (31) is programmed to automatically generate a fifth message directed to patient treatment personnel if the medical item information indicates that the medical item (38) is a DME item, the generation of the fifth message triggered by the medical item information contained in the fourth radio frequency signals becoming associated with the patient identifier, wherein the fifth message prompts patient treatment personnel to perform one or more of the following actions:
disclose information to the patient related to the proper use of a DME medical item (38); input information to verify delivery of a DME medical item (38) to the patient; and obtain the patient's signature to acknowledge receipt of a DME medical item (38).

13. The apparatus of claim 12, wherein the medical facility inventory computer (31) is programmed to automatically update records in the medical item inventory database (52) to transfer custody of the DME medical item (38) to the patient identified by the patient identifier upon obtaining the patient's signature entered on an input device in electrical communication with the medical facility inventory computer (31).

14. The apparatus of claim 8, wherein:
the one or more third RFID antennas (50a-50d) of the procedure room portal (48) are operable to receive radio frequency signals emanated from RFID tags attached to medical items (38) that were not used or consumed during the medical procedure (MP1) and which pass through the procedure room portal (48) as or before the medical items (38) leave the medical procedure room (PR1) to be returned to the supply room (68);
the third RFID reader (46) is for decoding the medical item information contained in the radio frequency signals emanated from the RFID tags; and
the medical facility inventory computer (31) is programmed to automatically associate a fifth personnel identifier with the medical item information contained in the radio frequency signals, wherein the association is triggered by the RFID tags on the medical items (38) leaving the medical procedure room (PR1) being detected by the third RFID reader (46), and wherein the fifth personnel identifier identifies a person responsible for the medical items (38) until the medical items (38) are returned to the supply room (68).

15. The apparatus of claim 14, further comprising:
a supply room portal having:
a portal opening; and
one or more second RFID antennas having fields of view directed to the portal opening, the one or more second RFID antennas for receiving second radio frequency signals emanated from RFID tags attached to medical items (38) that pass through the portal based on the medical items (38) being picked for removal from the supply room (68) to be used in the medical procedure (MP1); and
a second RFID reader electrically connected to the one or more second RFID antennas of the supply room portal, the second RFID reader for decoding the medical item information contained in the second radio frequency signals emanated from the RFID tags, wherein:
the one or more second RFID antennas of the supply room portal are operable to receive radio frequency signals emanated from RFID tags attached to medical items (38) that were not used or consumed during the medical procedure (MP1) and which pass through the supply room portal as or after the medical items (38) enter the supply room (68) to be returned to stock;
the second RFID reader is for decoding the medical item information contained in the radio frequency signals emanated from the RFID tags; and
the medical facility inventory computer (31) is programmed to automatically disassociate the fifth personnel identifier from the medical item information contained in the radio frequency signals, wherein the disassociation is triggered by the RFID tags on the medical items being detected by the second RFID reader.

## Patentansprüche

1. Vorrichtung zum Nachverfolgen der Obhut über Medizinprodukte (38) in einer Liefer- und Verbrauchskette, wobei an jedem Medizinprodukt (38) ein RFID-Etikett angebracht ist, das für das Medizinprodukt (38) spezifische Medizinproduktinformation kodiert, wobei die Medizinprodukte (38) anfänglich als eine Lieferung (54) von Medizinprodukten(38) zusammengefasst sind, und wobei mit der Lieferung (54) eine Packliste (56) verknüpft ist und mit der Packliste (56) eine Lieferungskennung (58) verknüpft ist, die für die Lieferung (54) spezifische Lieferungskenninformation kodiert, wobei die Vorrichtung umfasst:
eine erste Lesevorrichtung (59), die in einem Bereich einer medizinischen Einrichtung angeordnet ist, in dem die Lieferung (54) der Medizinprodukte (38) entgegengenommen wird, wobei die erste Lesevorrichtung (59) zum Lesen der der Packliste (56) zugeordneten Lieferungskennung (58) und zum Dekodieren der in der Lieferungskennung (58) kodierten Lieferungskenninformation dient;
ein oder mehrere erste RFID-Antennen (72), die in einem Vorratsraum (68) in der medizinischen Einrichtung angeordnet sind, wobei die ein oder mehreren ersten RFID-Antennen (72) zum Empfangen von ersten Hochfrequenzsignalen dienen, die von den an den Medizinprodukten (38) der Lieferung (54) angebrachten RFID-Etiketten abgegeben werden, nachdem die Medizinprodukte (38) in den Vorratsraum (68) gebracht worden sind, wobei die ersten Hochfrequenzsignale die in den an den Medizinprodukten(38) angebrachten RFID-Etiketten codierte Medizinproduktinformation enthalten;
ein oder mehrere erste RFID-Leser (66), die dem Vorratsraum (68) der medizinischen Einrichtung zugeordnet sind und elektrisch mit den ein oder mehreren ersten RFID-Antennen (72) verbunden sind, wobei die ein oder mehreren ersten RFID-Leser (66) zum Dekodieren der Medizinproduktinformation dienen, die in den von den an den Medizinprodukten(38) angebrachten RFID-Etiketten abgegebenen ersten Hochfrequenzsignalen enthalten ist;
eine zweite Lesevorrichtung (66), die dem Vorratsraum (68) der medizinischen Einrichtung zugeordnet ist, wobei die zweite Lesevorrichtung (66) zum Lesen zumindest der der Packliste (56) zugeordneten Lieferungskennung (58) und zum Dekodieren der in der Lieferungskennung (58) kodierten Lieferungskenninformation dient;
einen Inventarisierungscomputer (31) der medizinischen Einrichtung, der in elektrischer Kommunikationsverbindung mit dem ersten Lesegerät (59), den ein oder mehreren ersten RFID-Lesern (66) und der zweiten Lesevorrichtung (66) steht;
eine Medizinprodukt-Inventardatenbank (52), die in elektrischer Kommunikationsverbindung mit dem Inventarisierungscomputer (31) der medizinischen Einrichtung steht;
ein abgeschirmtes Gehäuse (12), das in einem medizinischen Behandlungsraum (PR1) angeordnet ist, wobei das abgeschirmte Gehäuse (12) umfasst:
einen Innenraum zur Aufnahme von Verpackungen (20) von Medizinprodukten(38), die während einer medizinischen Behandlung (MP1) verwendet werden;
eine Öffnung (24) in der Oberseite des abgeschirmten Gehäuses (12), durch die die Verpackungen (20) abgelegt werden; und
eine Abdeckung (25), die über der Öffnung (24) vorgesehen ist, um zu verhindern, dass RFID-Signale aus dem abgeschirmten Gehäuse (12) entweichen,
wobei das abgeschirmte Gehäuse (12) konfiguriert ist, um Hochfrequenzsignale, die von außerhalb des abgeschirmten Gehäuses (12) angeordneten RFID-Etiketten abgegeben werden, auf Pegel zu dämpfen, die in dem Innenraum im Wesentlichen nicht nachweisbar sind;
einen im Innenraum des abgeschirmten Gehäuses (12) angeordneten Abfallbehälter (18) zur Aufnahme von Verpackungen (20) von während der medizinischen Behandlung (MP1) verwendet Medizinprodukten (38);
ein oder mehrere vierte RFID-Antennen (14a, 14b), die in dem Innenraum des abgeschirmten Gehäuses (12) angeordnet sind, wobei die ein oder mehreren vierten RFID-Antennen (14a, 14b) zum Empfangen von vierten Hochfrequenzsignalen dienen, die von den RFID-Etiketten an den in dem Innenraum abgelegten Verpackungen (20) abgegeben werden, wobei die vierten Hochfrequenzsignale die in den RFID-Etiketten kodierte Medizinproduktinformation enthalten; und
mindestens einen vierten RFID-Leser (28), der elektrisch mit den ein oder mehreren vierten RFID-Antennen (14a, 14b) verbunden ist, wobei der mindestens eine vierte RFID-Leser (28) zum Dekodieren der Medizinproduktinformation dient, die in den von den RFID-Etiketten an den in dem Innenraum abgelegten Verpackungen (20) abgegebenen vierten Hochfrequenzsignalen enthalten ist, wobei:
der Inventarisierungscomputer (31) der medizinischen Einrichtung programmiert ist, der in den vierten Hochfrequenzsignalen enthaltenen Medizinproduktinformation automatisch eine Patientenkennung zuzuordnen, wobei die Zuordnung ausgelöst wird durch die Erfassung der RFID-Etiketten der Medizinprodukte (38) in der Lieferung (54) durch den vierten RFID-Leser (28),
die Patientenkennung einen Patienten identifiziert, an dem die medizinische Behandlung (MP1) in dem medizinischen Behandlungsraum (PR1) durchgeführt wurde;
der Inventarisierungscomputer (31) der medizinischen Einrichtung ferner programmiert ist zum Empfangen der Lieferungskenninformation, der Medizinproduktinformation, einer ersten Personenkennung (65), die eine Person identifiziert, die für die Entgegennahme der Lieferung (54) von Medizinprodukten (38) in der medizinischen Einrichtung verantwortlich ist, einer zweiten Personenkennung (69), die eine Person identifiziert, die für die Aufnahme der Medizinprodukte (38) in das Inventar in der medizinischen Einrichtung verantwortlich ist, und einer Vorratsraumkennung, die den Vorratsraum (68) identifiziert, in dem die Medizinprodukte (38) in das Inventar aufgenommen werden;
wobei die Medizinprodukt-Inventardatenbank (52) dazu dient, zumindest die Lieferungskennungsinformation mit der ersten Personenkennung (65), der zweiten Personenkennung (69) und der Vorratsraumkennung zu verknüpfen; und
der Inventarisierungscomputer (31) der medizinischen Einrichtung programmiert ist, um automatisch eine erste Nachricht, die an die für die Entgegennahme der Lieferung (54) von Medizinprodukten (38) in der medizinischen Einrichtung verantwortliche Person gerichtet ist, und/ oder eine zweite Nachricht, die an die für die Inventarisierung der Medizinprodukte (38) in der medizinischen Einrichtung zuständige Person gerichtet ist, zu erzeugen, wobei die Erzeugung der ersten Nachricht dadurch ausgelöst wird, dass die Lieferungskenninformation der ersten Personenkennung (65) zugeordnet wird, und die zweite Nachricht dadurch ausgelöst wird, dass die Lieferungskenninformation der zweiten Personenkennung (69) zugeordnet wird, wobei die erste und die zweite Nachricht die Person, an die sie gerichtet sind, auffordern, irgendeine Handlung in Bezug auf die Lieferung (54) von Medizinprodukten(38) vorzunehmen.

2. Vorrichtung nach Anspruch 1, bei der:
die Lieferungskennung (58) ein RFID-Etikett umfasst, das an der Packliste (56) angebracht ist, und wobei das erste Lesegerät (59) einen RFID-Leser umfasst;
das zweite Lesegerät (66) einen der ein oder mehreren dem Vorratsraum (68) zugeordneten ersten RFID-Leser (66) umfasst; und
der Inventarisierungscomputer (31) der medizinischen Einrichtung betreibbar ist, um die Vorratsraumkennung automatisch mit der Medizinproduktinformationen der Medizinprodukte (38) in der Lieferung (54) zu verknüpfen, wobei die Verknüpfung dadurch ausgelöst wird, dass die RFID-Etiketten an den Medizinprodukten (38) in der Lieferung (54) von den ein oder mehreren ersten RFID-Lesern (66) erfasst werden oder die Lieferungskennung (58) von dem zweiten Lesegerät (66) gelesen wird.

3. Vorrichtung nach Anspruch 1, bei der die Medizinprodukt-Inventardatenbank (52) eine Nachweiskette für die Medizinprodukte (38) in der Lieferung (54) aufrechterhält, die auf einem Abgleichzwischen der Lieferungskenninformation und der ersten Personenkennung (65) und der zweiten Personenkennung (69) basiert.

4. Vorrichtung nach Anspruch 1, die ferner umfasst:
ein Vorratsraumportal mit:
einer Portalöffnung; und
ein oder mehreren zweiten RFID-Antennen mit Sichtfeldern, die auf die Portalöffnung gerichtet sind, wobei die ein oder mehreren zweiten RFID-Antennen zum Empfangen von zweiten Hochfrequenzsignalen dienen, die von RFID-Etiketten abgegeben werden, die an Medizinprodukten (38) angebracht sind, die das Portal passieren, basierend auf den Medizinprodukten (38), die aufgenommen werden, um aus dem Vorratsraum (68) entnommen und in der medizinischen Behandlung (MP1) verwendet zu werden; und
einen zweiten RFID-Leser, der elektrisch mit den ein oder mehreren zweiten RFID-Antennen des Vorratsraumportals verbunden ist, wobei der zweite RFID-Leser zum Dekodieren der in den von den RFID-Etiketten ausgestrahlten zweiten Hochfrequenzsignalen enthaltenen Medizinproduktinformation dient, wobei:
der Inventarisierungscomputer (31) der medizinischen Einrichtung programmiert ist, automatisch eine dritte Personenkennung (75) mit der in den zweiten Hochfrequenzsignalen enthaltenen Medizinproduktinformation zu verknüpfen, wobei die Verknüpfung dadurch ausgelöst wird, dass die RFID-Etiketten an den Medizinprodukten(38) in der Lieferung (54) von dem zweiten RFID-Leser erfasst werden, und
wobei die dritte Personenkennung (75) eine Person identifiziert, die für die Entnahme der medizinischen Gegenstände (38) aus dem Vorratsraum (68) verantwortlich ist.

5. Vorrichtung nach Anspruch 4, bei der die Datenbank (52) für den Bestand an Medizinprodukteneine Verwahrkette für die Medizinprodukte (38) in der Lieferung (54) aufrechterhält, die auf einem Abgleich der in den zweiten Hochfrequenzsignalen enthaltene Medizinproduktinformation mit der dritten Personenkennung (75) beruht.

6. Vorrichtung nach Anspruch 4, bei der der Computer (31) der medizinischen Einrichtung betreibbar ist, um automatisch eine dritte Nachricht zu erzeugen, die an die Person gerichtet ist, die für die Entnahme der Medizinprodukte (38) aus dem Vorratsraum (68) verantwortlich ist, wobei die Erzeugung der dritten Nachricht dadurch ausgelöst wird, dass die in den zweiten Hochfrequenzsignalen enthaltene Medizinproduktinformation mit der dritten Personenkennung (75) verknüpft wird, wobei die dritte Nachricht die Person, an die sie gerichtet ist, auffordert, irgendeine Maßnahme in Bezug auf die aus dem Vorratsraum (68) entnommenen oder zu entnehmenden Medizinprodukte (38) zu treffen.

7. Vorrichtung nach Anspruch 4, bei der die zur Entnahme aus dem Vorratsraum (68) aufgenommenen medizinischen Gegenstände (38), die in der medizinischen Behandlung (MP1) verwendet werden sollen, in einem Transportbehälter (34) angeordnet sind, der ein daran angebrachtes RFID-Etikett (77) enthält, das Transportbehälter-Kennungsinformation codiert, wobei:
die ein oder mehreren zweiten RFID-Antennen des Versorgungsraumportals dazu dienen, ein Hochfrequenzsignal zu empfangen, das von dem an dem Transportbehälter (34) angebrachten RFID-Etikett (77) abgegeben wird, wenn dieser das Portal passiert;
der zweite RFID-Leser zum Dekodieren der Transportbehälter-Kennungsinformation dient, die in dem Hochfrequenzsignal enthalten ist, das von dem an dem Transportbehälter (34) angebrachten RFID-Etikett (77) ausgestrahlt wird; und
der Inventarisierungscomputer (31) der medizinischen Einrichtung programmiert ist, die Kennungsinformation des Transportbehälters automatisch mit der Medizinproduktinformation über die Medizinprodukte (38), die sich in dem Transportbehälter (34) befinden, zu verknüpfen.

8. Vorrichtung nach Anspruch 1, ferner umfassend:
ein Behandlungsraumportal (48) mit:
einer Portalöffnung (49); und
ein oder mehreren dritten RFID-Antennen (50a-50d) mit Sichtfeldern, die auf die Portalöffnung (49) gerichtet sind, wobei die ein oder mehreren dritten RFID-Antennen (50a-50d) zum Empfangen von dritten Hochfrequenzsignalen dienen, die von RFID-Etiketten ausgestrahlt werden, die an Medizinprodukten (38) angebracht sind, die das Portal (48) des Behandlungsraums passieren, wenn die medizinischen Gegenstände (38) in einen medizinischen Behandlungsraum (PR1) gebracht werden, um in der medizinischen Behandlung (MP1) verwendet zu werden; und
einen dritten RFID-Leser (46), der elektrisch mit den ein oder mehreren dritten RFID-Antennen (50a-50d) des Behandlungsraumportals (48) verbunden ist, wobei der dritte RFID-Leser (46) zum Dekodieren der Medizinproduktinformation dient, die in den von den RFID-Etiketten abgegebenen dritten Hochfrequenzsignalen enthalten ist, wobei:
der Inventarisierungscomputer (31) der medizinischen Einrichtung programmiert ist, automatisch eine vierte Personenkennung mit den in den dritten Hochfrequenzsignalen enthaltenen Medizinproduktinformation zu verknüpfen, wobei die Verknüpfung dadurch ausgelöst wird, dass die RFID-Etiketten an den Medizinprodukten(38) der Lieferung (54) von dem dritten RFID-Leser (46) erfasst werden, und
wobei die vierte Personenkennung eine Person identifiziert, die für die medizinischen Gegenstände (38) verantwortlich ist, während sich die medizinischen Gegenstände (38) in dem medizinischen Behandlungsraum (PR1) befinden.

9. Vorrichtung nach Anspruch 8, bei der die Medizinprodukt-Inventardatenbank (52) eine Verwahrkette für die medizinischen Gegenstände (38) in der Lieferung (54) aufrechterhält, die auf einem Abgleich der in den dritten Hochfrequenzsignalen enthaltenen Medizinproduktinformation mit der vierten Personenkennung beruht.

10. Vorrichtung nach Anspruch 8, bei der der Computer (31) der medizinischen Einrichtung betreibbar ist, um automatisch eine vierte Nachricht zu erzeugen, die an die Person gerichtet ist, die für die medizinischen Gegenstände (38) verantwortlich ist, während sich die medizinischen Gegenstände (38) in dem medizinischen Behandlungsraum (PR1) befinden, wobei die Erzeugung der vierten Nachricht dadurch ausgelöst wird, dass die in den dritten Hochfrequenzsignalen enthaltene Medizinproduktinformation mit der vierten Personenkennung verknüpft wird, wobei die vierte Nachricht die Person, an die sie gerichtet ist, auffordert, irgendeine Maßnahme in Bezug auf die in den medizinischen Behandlungsraum (PR1) gebrachten Medizinprodukte (38) zu treffen.

11. Vorrichtung nach Anspruch 1, bei der die Medizinprodukt-Inventardatenbank (52) eine Verwahrkette für die Medizinprodukte (38) der Lieferung (54) aufrechterhält, die auf einem Abgleich der in den vierten Hochfrequenzsignalen enthaltenen Medizinproduktinformation mit der Patientenkennung beruht.

12. Vorrichtung nach Anspruch 1, bei der der Inventarisierungscomputer (31) der medizinischen Einrichtung programmiert ist, automatisch eine fünfte Nachricht zu erzeugt, die an das Patientenbehandlungspersonal gerichtet ist, wenn die Medizinproduktinformation darauf hinweist, dass es sich bei dem Medizinprodukt (38) um ein DME-Produkt handelt, wobei die Erzeugung der fünften Nachricht dadurch ausgelöst wird, dass die in den vierten Hochfrequenzsignalen enthaltene Medizinproduktinformation mit der Patientenkennung verknüpft wird, wobei die fünfte Nachricht das Patientenbehandlungspersonal auffordert, eine oder mehrere der folgenden Maßnahmen zu treffen:
Offenbaren von Informationen an den Patienten, die sich auf die ordnungsgemäße Verwendung eines DME-Medizinprodukts beziehen (38); Eingeben von Informationen, um die Abgabe eines DME-Medizinprodukts (38) an den Patienten zu verifizieren; und Einholen der Unterschrift des Patienten zur Bestätigung des Empfangs eines DME-Medizinprodukts (38).

13. Vorrichtung nach Anspruch 12, bei der der Inventarisierungscomputer (31) der medizinischen Einrichtung programmiert ist, nach Empfang der in ein in elektrischer Verbindung mit dem Inventarisierungscomputer (31) der medizinischen Einrichtung stehendes Eingabegerät eingegebenen Unterschrift des Patienten automatisch die Aufzeichnungen in der Medizinprodukt-Inventardatenbank (52) zu aktualisieren, um die Obhut über das DME-Medizinprodukt (38) auf den durch die Patientenkennung identifizierten Patienten zu übertragen.

14. Vorrichtung nach Anspruch 8, bei der:
die ein oder mehreren dritten RFID-Antennen (50a- 50d) des Eingriffsraumportals (48) betreibbar sind, um Hochfrequenzsignale zu empfangen, die von RFID-Etiketten abgegeben werden, die an Medizinprodukten (38) angebracht sind, die während des medizinischen Eingriffs (MP1) nicht verwendet oder verbraucht wurden und die das Eingriffsraum portal (48) passieren, wenn oder bevor die Medizinprodukte (38) den medizinischen Behandlungsraum (PR1) verlassen, um in den Vorratsraum (68) zurückgebracht zu werden;
der dritte RFID-Leser (46) zum Dekodieren der in den von den RFID-Etiketten ausgestrahlten Hochfrequenzsignalen enthaltenen Medizinproduktinformation dient; und
der Inventarisierungscomputer (31) der medizinischen Einrichtung programmiert ist, automatisch eine fünfte Personenkennung mit der in den Hochfrequenzsignalen enthaltenen Medizinproduktinformation zu verknüpfen, wobei die Verknüpfung dadurch ausgelöst wird, dass die RFID-Etiketten an den Medizinprodukten (38), die den medizinischen Behandlungsraum (PR1) verlassen, von dem dritten RFID-Leser (46) erfasst werden, und wobei die fünfte Personenkennung eine Person identifiziert, die für die Medizinprodukte (38) verantwortlich ist, bis die Medizinprodukte (38) in den Vorratsraum (68) zurückgebracht sind.

15. Vorrichtung nach Anspruch 14, ferner umfassend
ein Vorratsraumportal mit:
einer Portalöffnung; und
einer oder mehreren zweiten RFID-Antennen mit Sichtfeldern, die auf die Portalöffnung gerichtet sind, wobei die ein oder mehreren zweiten RFID-Antennen zum Empfangen von zweiten Hochfrequenzsignalen dienen, die von RFID-Etiketten abgegeben werden, die an Medizinprodukten (38) angebracht sind, die das Portal passieren, basierend auf den Medizinprodukten (38), die aufgenommen werden, um aus dem Vorratsraum (68) entnommen und in der medizinischen Behandlung (MP1) verwendet zu werden; und
einen zweiten RFID-Leser, der elektrisch mit den ein oder mehreren zweiten RFID-Antennen des Vorratsraumportals verbunden ist, wobei der zweite RFID-Leser zum Dekodieren der in den von den RFID-Etiketten ausgestrahlten zweiten Hochfrequenzsignalen enthaltenen Medizinproduktinformation dient, wobei:
die ein oder mehreren zweiten RFID-Antennen des Vorratsraumportals betreibbar sind, um Hochfrequenzsignale zu empfangen, die von RFID-Etiketten abgegeben werden, die an Medizinprodukten (38) angebracht sind, die während der medizinischen Behandlung (MP1) nicht verwendet oder verbraucht wurden und die das Vorratsraumportal passieren, wenn oder nachdem die Medizinprodukte (38) in den Vorratsraum (68) eintreten, um in den Vorrat zurückgeführt zu werden;
der zweite RFID-Leser zum Dekodieren der Medizinproduktinformation dient, die in den von den RFID-Etiketten abgegebenen Hochfrequenzsignalen enthalten ist; und
der Inventarisierungscomputer (31) der medizinischen Einrichtung programmiert ist, automatisch die fünfte Personenkennung von der in den Hochfrequenzsignalen enthaltenen Medizinproduktinformation zu trennen, wobei die Trennung dadurch ausgelöst wird, dass die RFID-Etiketten an den Medizinprodukten durch den zweiten RFID-Leser erfasst werden.

## Revendications

1. Appareil pour suivre la garde d'articles médicaux (38) dans une chaîne d'approvisionnement et de consommation, où chaque article médical (38) a une étiquette RFID attachée à celui-ci qui code des informations d'article médical spécifiques à l'article médical (38), où les articles médicaux (38) sont initialement collectés ensemble sous forme d'un envoi (54) d'articles médicaux (38), et où l'envoi (54) a un bordereau d'expédition (56) associé à celui-ci et le bordereau d'expédition (56) a un identifiant d'envoi (58) associé à celui-ci qui code des informations d'identification d'envoi spécifiques à l'envoi (54), l'appareil comprenant :
un premier dispositif de lecture (59) disposé dans une zone d'un établissement médical à laquelle l'envoi (54) des articles médicaux (38) est reçu, le premier dispositif de lecture (59) étant destiné à lire l'identifiant d'envoi (58) associé au bordereau d'expédition (56) et à décoder les informations d'identification d'envoi codées dans l'identifiant d'envoi (58) ;
une ou plusieurs premières antennes RFID (72) disposées dans une salle d'approvisionnement (68) dans l'établissement médical, les une ou plusieurs premières antennes RFID (72) étant destinées à recevoir des premiers signaux radiofréquence émanant des étiquettes RFID attachées aux articles médicaux (38) dans l'envoi (54) après que les articles médicaux (38) ont été amenés dans la salle d'approvisionnement (68), où les premiers signaux radiofréquence contiennent les informations d'article médical codées dans les étiquettes RFID attachées aux articles médicaux (38) ;
un ou plusieurs premiers lecteurs RFID (66) associés à la salle d'approvisionnement (68) de l'établissement médical et reliés électriquement aux une ou plusieurs premières antennes RFID (72), les un ou plusieurs premiers lecteurs RFID (66) étant destinés à décoder les informations d'article médical contenues dans les premiers signaux radiofréquence émanant des étiquettes RFID attachées aux articles médicaux (38) ;
un deuxième dispositif de lecture (66) associé à la salle d'approvisionnement (68) de l'établissement médical, le deuxième dispositif de lecture (66) étant destiné à lire au moins l'identifiant d'envoi (58) associé au bordereau d'expédition (56) et à décoder les informations d'identification d'envoi codées dans l'identifiant d'envoi (58) ;
un ordinateur d'inventaire d'établissement médical (31) en communication électrique avec le premier dispositif de lecture (59) ; les un ou plusieurs premiers lecteurs RFID (66) ; et le deuxième dispositif de lecture (66) ;
une base de données d'inventaire d'articles médicaux (52) en communication électrique avec l'ordinateur d'inventaire d'établissement médical (31) ;
une enceinte blindée (12) disposée dans une salle d'intervention médicale (PR1), l'enceinte blindée (12) comprenant :
un espace interne destiné à recevoir des emballages (20) d'articles médicaux (38) utilisés lors d'une intervention médicale (MP1) ;
une ouverture (24) dans la partie supérieure de l'enceinte blindée (12) à travers laquelle les emballages (20) sont déposés ; et
un couvercle (25) prévu au-dessus de l'ouverture (24) pour empêcher des signaux RFID de s'échapper de l'enceinte blindée (12),
l'enceinte blindée (12) étant configurée pour atténuer des signaux radiofréquence émanant d'étiquettes RFID disposées à l'extérieur de l'enceinte blindée (12) jusqu'à des niveaux qui sont sensiblement indétectables dans l'espace interne ;
un bac à déchets (18) destiné à recevoir des emballages (20) d'articles médicaux (38) utilisés lors de l'intervention médicale (MP1) disposé dans l'espace interne de l'enceinte blindée (12) ;
une ou plusieurs quatrièmes antennes RFID (14a, 14b) disposées dans l'espace interne de l'enceinte blindée (12), les une ou plusieurs quatrièmes antennes RFID (14a, 14b) étant destinées à recevoir des quatrièmes signaux radiofréquence émanant d'étiquettes RFID attachées aux emballages (20) disposés dans l'espace interne, où les quatrièmes signaux radiofréquence contiennent les informations d'article médical codées dans les étiquettes RFID ; et
au moins un quatrième lecteur RFID (28) relié électriquement aux une ou plusieurs quatrièmes antennes RFID (14a, 14b), l'au moins un quatrième lecteur RFID (28) étant destiné à décoder les informations d'article médical contenues dans les quatrièmes signaux radiofréquence émanant des étiquettes RFID attachées aux emballages (20) disposés dans l'espace interne, où :
l'ordinateur d'inventaire d'établissement médical (31) est programmé pour associer automatiquement un identifiant de patient aux informations d'article médical contenues dans les quatrièmes signaux radiofréquence, où l'association est déclenchée par les étiquettes RFID des articles médicaux (38) dans l'envoi (54) qui sont détectées par le quatrième lecteur RFID (28), et où l'identifiant de patient identifie un patient sur lequel l'intervention médicale (MP1) a été réalisée dans la salle d'intervention médicale (PR1) ;
l'ordinateur d'inventaire d'établissement médical (31) est en outre programmé pour recevoir les informations d'identification d'envoi, les informations d'article médical, un premier identifiant de personnel (65) qui identifie une personne responsable de la réception de l'envoi (54) d'articles médicaux (38) au niveau de l'établissement médical, un deuxième identifiant de personnel (69) qui identifie une personne responsable du placement des articles médicaux (38) dans l'inventaire au niveau de l'établissement médical, et un identifiant de salle d'approvisionnement qui identifie la salle d'approvisionnement (68) dans laquelle les articles médicaux (38) sont placés dans l'inventaire ;
la base de données d'inventaire d'articles médicaux (52) est destinée à associer au moins les informations d'identification d'envoi au premier identifiant de personnel (65), au deuxième identifiant de personnel (69) et à l'identifiant de salle d'approvisionnement ; et
l'ordinateur d'inventaire d'établissement médical (31) est programmé pour générer automatiquement l'un ou les deux parmi un premier message adressé à la personne responsable de la réception de l'envoi (54) d'articles médicaux (38) au niveau de l'établissement médical et un deuxième message adressé à la personne responsable du placement des articles médicaux (38) dans l'inventaire au niveau de l'établissement médical, la génération du premier message étant déclenchée par les informations d'identification d'envoi devenant associées au premier identifiant de personnel (65), et la génération du deuxième message étant déclenchée par les informations d'identification d'envoi devenant associées au deuxième identifiant de personnel (69), où les premier et deuxième messages invitent la personne à qui ils sont adressés à prendre certaines mesures concernant l'envoi (54) d'articles médicaux (38).

2. Appareil de la revendication 1, dans lequel :
l'identifiant d'envoi (58) comprend une étiquette RFID attachée au bordereau d'expédition (56), et où le premier dispositif de lecture (59) comprend un lecteur RFID ;
le deuxième dispositif de lecture (66) comprend l'un des un ou plusieurs premiers lecteurs RFID (66) associés à la salle d'approvisionnement (68) ; et
l'ordinateur d'inventaire d'établissement médical (31) a pour fonction d'associer automatiquement l'identifiant de salle d'approvisionnement aux informations d'article médical des articles médicaux (38) dans l'envoi (54), où l'association est déclenchée par les étiquettes RFID sur les articles médicaux (38) dans l'envoi (54) qui sont détectées par les un ou plusieurs premiers lecteurs RFID (66) ou l'identifiant d'envoi (58) qui est lu par le deuxième dispositif de lecture (66).

3. Appareil de la revendication 1, dans lequel la base de données d'inventaire d'articles médicaux (52) maintient une chaîne de garde des articles médicaux (38) dans l'envoi (54) sur la base d'une référence croisée des informations d'identification d'envoi avec le premier identifiant de personnel (65) et le deuxième identifiant de personnel (69) .

4. Appareil de la revendication 1, comprenant en outre :
un portail de salle d'approvisionnement ayant :
une ouverture de portail ; et
une ou plusieurs deuxièmes antennes RFID ayant des champs de vision dirigés vers l'ouverture de portail, les une ou plusieurs deuxièmes antennes RFID étant destinées à recevoir des deuxièmes signaux radiofréquence émanant d'étiquettes RFID attachées à des articles médicaux (38) qui traversent le portail sur la base des articles médicaux (38) qui sont sélectionnés pour être retirés de la salle d'approvisionnement (68) pour être utilisés dans l'intervention médicale (MP1) ; et
un deuxième lecteur RFID relié électriquement aux une ou plusieurs deuxièmes antennes RFID du portail de salle d'approvisionnement, le deuxième lecteur RFID étant destiné à décoder les informations d'article médical contenues dans les deuxièmes signaux radiofréquence émanant des étiquettes RFID, où :
l'ordinateur d'inventaire d'établissement médical (31) étant programmé pour associer automatiquement un troisième identifiant de personnel (75) aux informations d'article médical contenues dans les deuxièmes signaux radiofréquence, où l'association est déclenchée par les étiquettes RFID sur les articles médicaux (38) dans l'envoi (54) qui sont détectées par le deuxième lecteur RFID, et où le troisième identifiant de personnel (75) identifie une personne responsable du retrait des articles médicaux (38) de la salle d'approvisionnement (68).

5. Appareil de la revendication 4, dans lequel la base de données d'inventaire d'articles médicaux (52) maintient une chaîne de garde des articles médicaux (38) dans l'envoi (54) sur la base d'une référence croisée des informations d'article médical contenues dans les deuxièmes signaux radiofréquence avec le troisième identifiant de personnel (75) .

6. Appareil de la revendication 4, dans lequel l'ordinateur d'inventaire d'établissement médical (31) a pour fonction de générer automatiquement un troisième message adressé à la personne responsable du retrait des articles médicaux (38) de la salle d'approvisionnement (68), la génération du troisième message étant déclenchée par les informations d'article médical contenues dans les deuxièmes signaux radiofréquence devenant associées au troisième identifiant de personnel (75), où le troisième message invite la personne à qui il est adressé à prendre certaines mesures concernant les articles médicaux (38) qui ont été retirés ou qui doivent être retirés de la salle d'approvisionnement (68).

7. Appareil de la revendication 4, dans lequel les articles médicaux (38) qui ont été sélectionnés pour être retirés de la salle d'approvisionnement (68) pour être utilisés dans l'intervention médicale (MP1), sont disposés dans un conteneur de transport (34) qui comporte une étiquette RFID (77) attachée à celui-ci qui code des informations d'identification de conteneur de transport,
dans lequel :
les une ou plusieurs deuxièmes antennes RFID du portail de salle d'approvisionnement sont destinées à recevoir un signal radiofréquence émanant de l'étiquette RFID (77) attachée au conteneur de transport (34) à mesure qu'il traverse le portail ;
le deuxième lecteur RFID est destiné à décoder les informations d'identification de conteneur de transport contenues dans le signal radiofréquence émanant de l'étiquette RFID (77) attachée au conteneur de transport (34) ; et
l'ordinateur d'inventaire d'établissement médical (31) est programmé pour associer automatiquement les informations d'identification de conteneur de transport aux informations d'article médical des articles médicaux (38) disposés dans le conteneur de transport (34).

8. Appareil de la revendication 1, comprenant en outre :
un portail de salle d'intervention (48) ayant :
une ouverture de portail (49) ; et
une ou plusieurs troisièmes antennes RFID (50a-50d) ayant des champs de vision dirigés vers l'ouverture de portail (49), les une ou plusieurs troisièmes antennes RFID (50a-50d) étant destinées à recevoir des troisièmes signaux radiofréquence émanant d'étiquettes RFID attachées à des articles médicaux (38) qui traversent le portail de salle d'intervention (48) lorsque les articles médicaux (38) sont amenés dans une salle d'intervention médicale (PR1) pour être utilisés dans l'intervention médicale (MP1) ; et
un troisième lecteur RFID (46) relié électriquement aux une ou plusieurs troisièmes antennes RFID (50a-50d) du portail de salle d'intervention (48), le troisième lecteur RFID (46) étant destiné à décoder les informations d'article médical contenues dans les troisièmes signaux radiofréquence émanant des étiquettes RFID,
dans lequel :
l'ordinateur d'inventaire d'établissement médical (31) est programmé pour associer automatiquement un quatrième identifiant de personnel aux informations d'article médical contenues dans les troisièmes signaux radiofréquence, où l'association est déclenchée par les étiquettes RFID sur les articles médicaux (38) dans l'envoi (54) qui sont détectées par le troisième lecteur RFID (46), et où le quatrième identifiant de personnel identifie une personne responsable des articles médicaux (38) pendant que les articles médicaux (38) se trouvent dans la salle d'intervention médicale (PR1).

9. Appareil de la revendication 8, dans lequel la base de données d'inventaire d'articles médicaux (52) maintient une chaîne de garde des articles médicaux (38) dans l'envoi (54) sur la base d'une référence croisée des informations d'article médical contenues dans les troisièmes signaux radiofréquence avec le quatrième identifiant de personnel.

10. Appareil de la revendication 8, dans lequel l'ordinateur d'inventaire d'établissement médical (31) a pour fonction de générer automatiquement un quatrième message adressé à la personne responsable des articles médicaux (38) pendant que les articles médicaux (38) se trouvent dans la salle d'intervention médicale (PR1), la génération du quatrième message étant déclenchée par les informations d'article médical contenues dans les troisièmes signaux radiofréquence devenant associées au quatrième identifiant de personnel, où le quatrième message invite la personne à qui il est adressé à prendre certaines mesures concernant les articles médicaux (38) amenés dans la salle d'intervention médicale (PR1).

11. Appareil de la revendication 1, dans lequel la base de données d'inventaire d'articles médicaux (52) maintient une chaîne de garde des articles médicaux (38) dans l'envoi (54) sur la base d'une référence croisée des informations d'article médical contenues dans les quatrièmes signaux radiofréquence avec l'identifiant de patient.

12. Appareil de la revendication 1, dans lequel l'ordinateur d'inventaire d'établissement médical (31) est programmé pour générer automatiquement un cinquième message adressé à un personnel chargé du traitement de patient si les informations d'article médical indiquent que l'article médical (38) est un article DME (pour « Durable Medical Equipment », matériel médical non consommable), la génération du cinquième message étant déclenchée par les informations d'article médical contenues dans les quatrièmes signaux radiofréquence devenant associées à l'identifiant de patient, où le cinquième message invite un personnel chargé du traitement de patient à réaliser une ou plusieurs des actions suivantes :
la divulgation au patient d'informations relatives à la bonne utilisation d'un article médical DME (38) ; l'entrée d'informations pour vérifier la livraison d'un article médical DME (38) au patient ; et l'obtention de la signature du patient pour accuser réception d'un article médical DME (38).

13. Appareil de la revendication 12, dans lequel l'ordinateur d'inventaire d'établissement médical (31) est programmé pour mettre à jour automatiquement des enregistrements dans la base de données d'inventaire d'articles médicaux (52) pour transférer la garde de l'article médical DME (38) au patient identifié par l'identifiant de patient lors de l'obtention de la signature du patient introduite sur un dispositif d'entrée en communication électrique avec l'ordinateur d'inventaire d'établissement médical (31).

14. Appareil de la revendication 8, dans lequel :
les une ou plusieurs troisièmes antennes RFID (50a-50d) du portail de salle d'intervention (48) ont pour fonction de recevoir des signaux radiofréquence émanant d'étiquettes RFID attachées à des articles médicaux (38) qui n'ont pas été utilisés ou consommés lors de l'intervention médicale (MP1) et qui traversent le portail de salle d'intervention (48) à mesure que ou avant que les articles médicaux (38) quittent la salle d'intervention médicale (PR1) pour être retournés à la salle d'approvisionnement (68) ;
le troisième lecteur RFID (46) est destiné à décoder les informations d'article médical contenues dans les signaux radiofréquence émanant des étiquettes RFID ; et
l'ordinateur d'inventaire d'établissement médical (31) est programmé pour associer automatiquement un cinquième identifiant de personnel aux informations d'article médical contenues dans les signaux radiofréquence, où l'association est déclenchée par les étiquettes RFID sur les articles médicaux (38) quittant la salle d'intervention médicale (PR1) qui sont détectées par le troisième lecteur RFID (46), et où le cinquième identifiant de personnel identifie une personne responsable des articles médicaux (38) jusqu'à ce que les articles médicaux (38) soient retournés à la salle d'approvisionnement (68).

15. Appareil de la revendication 14, comprenant en outre :
un portail de salle d'approvisionnement ayant :
une ouverture de portail ; et
une ou plusieurs deuxièmes antennes RFID ayant des champs de vision dirigés vers l'ouverture de portail, les une ou plusieurs deuxièmes antennes RFID étant destinées à recevoir des deuxièmes signaux radiofréquence émanant d'étiquettes RFID attachées à des articles médicaux (38) qui traversent le portail sur la base des articles médicaux (38) qui sont sélectionnés pour être retirés de la salle d'approvisionnement (68) pour être utilisés dans l'intervention médicale (MP1) ; et
un deuxième lecteur RFID relié électriquement aux une ou plusieurs deuxièmes antennes RFID du portail de salle d'approvisionnement, le deuxième lecteur RFID étant destiné à décoder les informations d'article médical contenues dans les deuxièmes signaux radiofréquence émanant des étiquettes RFID, où :
les une ou plusieurs deuxièmes antennes RFID du portail de salle d'approvisionnement ont pour fonction de recevoir des signaux radiofréquence émanant d'étiquettes RFID attachées à des articles médicaux (38) qui n'ont pas été utilisés ou consommés lors de l'intervention médicale (MP1) et qui traversent le portail de salle d'approvisionnement à mesure que ou après que les articles médicaux (38) entrent dans la salle d'approvisionnement (68) pour être retournés au stock ;
le deuxième lecteur RFID est destiné à décoder les informations d'article médical contenues dans les signaux radiofréquence émanant des étiquettes RFID ; et
l'ordinateur d'inventaire d'établissement médical (31) est programmé pour dissocier automatiquement le cinquième identifiant de personnel des informations d'article médical contenues dans les signaux radiofréquence, où la dissociation est déclenchée par les étiquettes RFID sur les articles médicaux qui sont détectées par le deuxième lecteur RFID.
